# EUROPEAN PATENT SPECIFICATION

(11) **EP 4 370 147 B1**
(45) Date of publication and mention of the grant of the patent: **18.11.2026**
(21) Application number: 22748085.2
(22) Date of filing: 14.07.2022
(51) Int. Cl.: A61K 38/39, A23L 2/39, A23L 33/18, A61K 8/65, A61P 13/10, A61P 15/02, A61P 17/00, A61P 17/02, A61P 19/02, A61Q 19/00, C07K 14/78

(54) **COLLAGEN POWDER, PROCESS FOR ITS PREPARATION AND USES THEREOF**
KOLLAGENPULVER, VERFAHREN ZU SEINER HERSTELLUNG UND SEINE VERWENDUNG
POUDRE DE COLLAGÈNE, PROCÉDÉ POUR SA PRÉPARATION ET SES UTILISATIONS

(30) Priority: 14.07.2021 IT 202100018647
(43) Date of publication of application: 22.05.2024
(73) Proprietor: Typeone Biomaterials S.r.l., 73036 Muro Leccese (LE) (IT)
(72) Inventor: SALVATORE, Luca, 73036 Muro Leccese (LE) (IT); LAURENZA, Massimo, 73036 Muro Leccese (LE) (IT)
(74) Representative: Gislon, Gabriele
(86) International application number: PCT/IB2022/056508
(87) International publication number: WO 2023/286012

(56) References cited:
- WO-A2-2020/060761
- LEÓN-LÓPEZ ARELY ET AL: "Hydrolyzed Collagen-Sources and Applications", vol. 24, no. 22, 7 November 2019 (2019-11-07), pages 4031, XP055903004, Retrieved from the Internet <URL:https://www.ncbi.nlm.nih.gov/labs/pmc/articles/PMC6891674/pdf/molecules-24-04031.pdf> DOI: 10.3390/molecules24224031
- MOSKOWITZ R W ED - BREEDVELD FERDINAND CHRISTOFFEL: "ROLE OF COLLAGEN HYDROLYSATE IN BONE AND JOINT DISEASE", SEMINARS IN ARTHRITIS AND RHEUMATISM, ELSEVIER, AMSTERDAM, NL, vol. 30, no. 2, 1 October 2000 (2000-10-01), pages 87 - 99, XP009021767, ISSN: 0049-0172, DOI: 10.1053/SARH.2000.9622
- DATABASE GNPD [online] MINTEL; 27 April 2021 (2021-04-27), ANONYMOUS: "Original Hydrolyzed Collagen Powder", XP055902994, retrieved from https://www.gnpd.com/sinatra/recordpage/8657549/ Database accession no. 8657549
- MATHEW-STEINER SHOMITA S. ET AL: "Collagen in Wound Healing", vol. 8, no. 5, 1 January 2021 (2021-01-01), pages 63, XP055903006, Retrieved from the Internet <URL:https://www.ncbi.nlm.nih.gov/labs/pmc/articles/PMC8151502/pdf/bioengineering-08-00063.pdf> DOI: 10.3390/bioengineering8050063
- SUGIHARA FUMIHITO ET AL: "Ingestion of bioactive collagen hydrolysates enhanced pressure ulcer healing in a randomized double-blind placebo-controlled clinical study", vol. 8, no. 1, 1 December 2018 (2018-12-01), pages 11403, XP055903007, Retrieved from the Internet <URL:https://www.ncbi.nlm.nih.gov/labs/pmc/articles/PMC6065362/pdf/41598_2018_Article_29831.pdf> DOI: 10.1038/s41598-018-29831-7
- DATABASE GNPD [online] MINTEL; 12 May 2021 (2021-05-12), ANONYMOUS: "Hydrolyzed Collagen Sachets", XP055902997, retrieved from https://www.gnpd.com/sinatra/recordpage/8701009/ Database accession no. 8701009

## Description

### Summary of the invention

The present invention relates to a hydrolyzed equine type I collagen powder having specific characteristics and uses thereof, in therapy.

### Background art

Collagen is the predominant protein in the connective tissue of animals, including humans, and is the most abundant protein in mammals (about 25% of total protein mass), constituting about 6% of body weight in humans, being found in all supporting structures such as ligaments, muscles, joints, synovial membranes, cartilage, bone, and skin.

Collagen is composed of fibers oriented parallel to each other so as to guarantee good mechanical support as well as high tensile strength.

There are several types of collagen; "type I" collagen accounts for 90% of total collagen and is involved in the composition of the major connective tissues, such as skin, tendons, bones and cornea. The structural unit of collagen is represented by tropocollagen, which is a protein of about 285 kDa formed by three polypeptide chains with a left-handed course that are combined to form a right-handed triple helix.

As age increases, the amount of collagen in body tissues decreases, while various diseases and some traumas can change the composition of the collagen fibers, thus causing connective tissues to degenerate and leading to reduced tensile strength. Synovial membranes and tendons become fragile, less tensile-strength resistant, and cartilage becomes thinner or even completely disappears. Collagen administrations and infiltrations can slow this process and, in some cases, accelerate post-injury regeneration. Collagen infiltrations are applied in case of muscle injuries and injuries of the joint ligament system (e.g., muscle strains or ligament sprains), in joint, vertebra and tendon degeneration processes, e.g., in tendinitis.

Various supplements, creams and powders containing collagen are commercially available for a variety of uses, from pharmaceutical use to food use. However, there is still a need for providing new collagen-based formulations that are versatile and allow the use in a wide range of applications.

WO 2020/060761 A2 discloses a multi-component oral supplement comprising, inter alia, hydrolyzed porcine collagen in powder form passing an 80-mesh sieve (corresponding to a particle size of approximately 180 µm), together with hyaluronic acid and glucosamine, for use in the treatment of degenerative joint disease. However, that document discloses neither a hydrolyzed equine type I collagen nor the specific particle-size and molecular-weight distributions of the present collagen powder.

### Objects of the invention

It is an object of the invention to provide a new hydrolyzed equine type I collagen powder having a specific particle size, said collagen consisting of oligomers having specific molecular weights.

It is another object of the invention to provide pharmaceutical and nutraceutical compositions containing said collagen powder.

It is another object of the invention to provide medical devices for administering said collagen powder and/or compositions containing the same.

It is a further object of the invention to provide the use in therapy of said collagen powder and/or pharmaceutical compositions containing the same.

Finally, it is a further object of the invention to provide the nutraceutical and/or cosmetic non-therapeutic use of the collagen powder of the invention.

### Brief description of the drawings

Figure 1 shows the particle size distribution of the collagen powder of the Example 1 as a logarithmic (bell-shaped curve) and cumulative % distribution.
Figure 2 shows the experimental numerical data of the particle size distribution in Figure 1.
Figure 3 shows the structured approach to select a sample preparation method [Excerpt from ISO 10993-3: 2014].

### Description of the Invention

- The present invention is defined in the appended claims.

According to one of its aspects, subject-matter of the invention is a hydrolyzed equine type I collagen powder (hereinafter also just "collagen powder") having the following particle size distribution:
- at most 10% of the particles have a mean diameter (d0.1) lower than 2.5 microns;
- at least 50% of the particles have a mean diameter (d0.5) lower than 10 microns;
- at least 90% of the particles have a mean diameter (d0.9) lower than 20 microns;
and
it consists of oligopeptides having the following molecular weights:
- at least 30% of the oligopeptide molecules have a weight average molecular weight from 80 to 120 kDa;
- at least 25% of the oligopeptide molecules have a weight average molecular weight from 50 to 70 kDa;
- at least 25% of the oligopeptide molecules have a weight average molecular weight from 25 to 45 kDa;
- at least 85% of said collagen has a molecular weight from 25 to 120 kDa.

Mean diameter herein means the mean diameter of the single collagen particle obtained by the process described below, whose shape is, to a first approximation, comparable to a sphere.

According to a preferred embodiment, in the collagen powder of the invention, 100% of the particles have a mean diameter from 0.5 to 50 microns, preferably 0.6 to 40 microns.

According to a preferred embodiment, in the collagen powder of the invention having the particle size set forth above, the collagen oligopeptides are characterized by the following molecular weights:
- at least 30% of the oligopeptide molecules have a weight average molecular weight from 80 to 120 kDa;
- at least 25% of the oligopeptide molecules have a weight average molecular weight from 50 to 70 kDa;
- at least 25% of the oligopeptide molecules have a weight average molecular weight from 25 to 45 kDa;
- at least 14.5% of the oligopeptide molecules have a weight average molecular weight from 100 to 110 kDa;
- at least 11.5% of the oligopeptide molecules have a weight average molecular weight from 80 to 90 kDa;
- at least 8.0% of the oligopeptide molecules have a weight average molecular weight from 40 to 45kDa;
- at least 85% of said collagen has a molecular weight from 25 to 120 kDa.

According to a preferred embodiment, in the collagen powder of the invention having the particle size set forth above, the collagen oligopeptides are characterized by the following molecular weights:
- at least 3.0% of the oligopeptide molecules have a weight average molecular weight of about 120 kDa;
- at least 14.5% of the oligopeptide molecules have a weight average molecular weight from 100 to 110 kDa;
- at least 11.5% of the oligopeptide molecules have a weight average molecular weight from 80 to 90 kDa;
- at least 7.0% of the oligopeptide molecules have a weight average molecular weight of about 70 kDa;
- at least 8.0% of the oligopeptide molecules have a weight average molecular weight of about 60 kDa;
- at least 10.5% of the oligopeptide molecules have a weight average molecular weight of about 50 kDa;
- at least 8.0% of the oligopeptide molecules have a weight average molecular weight from 40 to 45kDa;
- at least 9.5% of the oligopeptide molecules have a weight average molecular weight of about 35kDa;
- at least 6.5% of the oligopeptide molecules have a weight average molecular weight of about 25kDa;
- at least 85% of said collagen has a molecular weight between 25 and 120kDa.

Molecular weights were determined by protein electrophoresis. Protein electrophoresis is a commonly used analytical technique for the analysis of protein extracts and their integrity. The equipment and conditions used to evaluate the molecular weights by protein electrophoresis are set forth in the Experimental Section below.

According to a preferred embodiment, the hydrolyzed collagen powder of the invention is produced by proteolysis from atelocollagen, which in turn is derived from the removal of the telopeptide terminals of the type I collagen. The collagen of the invention is of equine origin. Preferably, the equine biological tissue the collagen is extracted from is the tendon, particularly the flexor tendon.

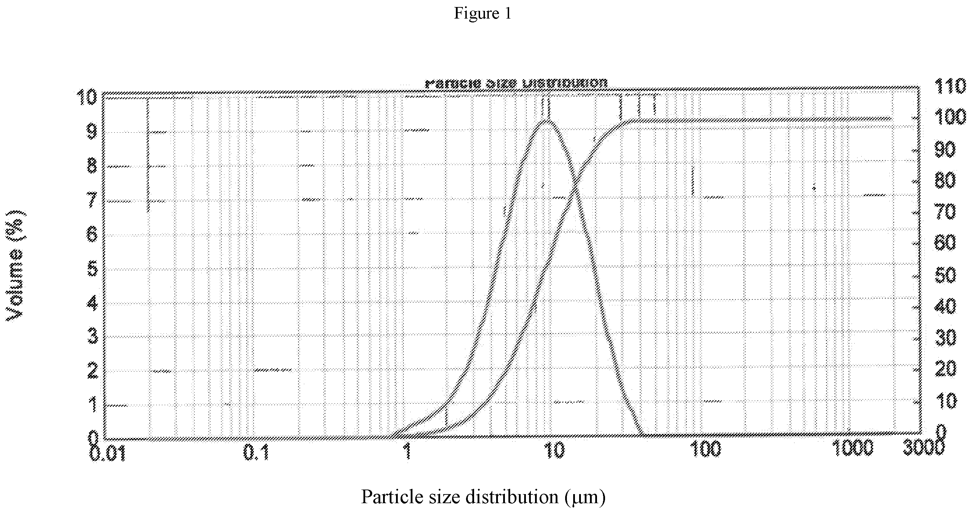

According to a preferred embodiment, the collagen powder of the invention shows the particle size distribution of Figure 1.

The collagen of the invention may be prepared by a process that comprises:
(i) suspending collagen of type I, deprived of the telopeptide fractions, in acidified water and subjecting it to proteolysis; and
(ii) drying said collagen in a micronized form.

In step (i), concentrated acid suspensions of collagen having weight concentrations from 3% to 5% (w/v) collagen, are incubated with proteolytic enzymes, such as pepsin, in order to modulate the molecular weights of the collagen polypeptide chains. At this stage, collagen macromolecules (i.e., triple-helices) are partially unstructured and opened. This treatment increases bioavailability, bioabsorption thereof. Next, the suspension is sprayed in the form of aerosol, which is then rapidly dried so as to obtain a powder; this process is known in industrial practice as "spray-drying."

By way of example, the proteolysis can be carried out by using an acid solution, such as acetic acid in demineralized water, at pH between 3.0 and 4.0, containing 1.0-10.0%, w/w pepsin (wt pepsin/wt collagen) at a temperature of about 37°C, for a few hours, such as 2-24 hours.

Other methods can alternatively be used to produce the collagen powder of the invention.

The collagen powder of the invention has proven to be particularly effective in a variety of uses, such as for example as a supplement, in cosmetics and in the medical field. In particular, its characteristic molecular weight distribution has been observed to promote excellent regeneration at the dermal and mucosal levels.

For its use, the collagen powder of the invention can be used as it is or, if necessary, after appropriate sterilization treatment (e.g., by using β or γ irradiation, according to known techniques). For example, the collagen powder of the invention can be sprayed or applied to wounds, burns, sores or the like, or alternatively it can be formulated into a pharmaceutical or nutraceutical or cosmetic composition, or included in medical devices.

"Pharmaceutical, nutraceutical or cosmetic composition" means a composition comprising the collagen powder of the invention in association with at least one carrier acceptable for the use such composition is intended for. A "pharmaceutically acceptable carrier" includes diluents, preservative agents, fillers, disintegrating agents, wetting agents, emulsifying agents, suspending agents, sweetening agents, flavoring agents, perfuming agents, antibacterial agents, antifungal agents, lubricating agents, and dispensing agents. Descriptions of pharmaceutically acceptable carriers and factors involved in their selection are known in the art.

Depending on the use the composition of the invention is intended for, it may be liquid or solid, or may be formulated into gels, creams, ointments, pastes and the like, which may also be sterile, if required, such as in the case of injectable preparations or preparations to be used on open wounds.

As stated, the collagen powder of the invention can also be used in combination with medical devices, such as, but not limited to, medicated patches and gauzes for the treatment of wounds, burns and sores of any nature, or it can be packaged as sterile solution in pre-filled syringes.

The use of the collagen powder of the invention, or compositions containing it, in therapy, in cosmetics and as dietary supplement constitutes further subject-matter of the invention.

By way of example, the collagen powder of the invention can be used for the treatment of wounds and sores, by direct application or through medical devices, or it can be used for the treatment of joint disorders, such as by infiltration, for the treatment of interstitial cystitis, such as by injection of viscous and/or adhesive compositions, for vaginal application, such as in the treatment of vulvovaginitis, etc.

The collagen powder of the invention can also be used for the regeneration and bio-revitalization of dermis and for the visco-supplementation and regeneration in the synovia in cases of osteo-chondral damage.

In fact, the collagen powder of the invention is very useful for preventing and treating skin aging and can be administered in form of creams, lotions, gels, and the like or injected under the skin as filler, for example, but not limited to, for wrinkle smoothing or for solving other aesthetic problems.

As stated, the collagen powder of the invention can also be taken orally as a dietary supplement or be included in cosmetic compositions.

The composition of the invention may comprise, in addition to the powder of the invention and any appropriate carriers and additives, other active ingredients, such as hyaluronic acid or salts thereof, e.g., sodium hyaluronate, of various molecular weights, chondroitin or salts thereof, e.g., chondroitin sulfate, probiotics, prebiotics, postbiotics, vitamins, such as Vitamin C, antimicrobial agents such as silver, and the like.

According to another of its aspects, the subject-matter of the invention is a method to treat sores, burns or wounds, joint disorders, aging skin, interstitial cystitis and vulvovaginitis, which includes administering an effective amount of the powder or composition of the invention to an individual in need thereof.

The individual receiving the powder or composition of the invention is generally a mammal, including, but not limited to, human beings.

The invention will now be described in detail in the following examples for illustrative purposes and in no way limiting.

### Experimental section

The equipment and conditions of the particle size analysis are set forth below:

| | |
|---|---|
| Material: | Collagen powder |
| Equipment: | Hydro 2000S (A) |
| Refractive index: | 1.6 |
| Absorption: | 0.01 |
| Size range: | 0.02-2000um |
| Dispersant: | Tegiloxan |
| Stirring speed: | 2800rpm |
| Concentration: | 0.0171 % Vol |
| Span: | 1,778 |
| Unit: | Volume |

The equipment and conditions to evaluate the molecular weights by protein electrophoresis are set forth below:
Mini-Protean Tetra Cell electrophoretic apparatus (BioRad Laboratories, Inc.) with polyacrylamide gel. The polyacrylamide gel was prepared manually (8% separation gel, 5% concentration gel) by using an acrylamide/bisacrylamide solution with a ratio of 37.5:1. Samples of about 5 mg were suspended in 0.2 ml of 0.5 M acetic acid. Next, a reducing solution (0.1 ml) consisting of Laemmli buffer (62.5 mM Tris-HCl pH 6.8, 10% glycerol, 2% SDS, 0.01% bromophenol blue, 5% β-mercaptoethanol) and 2M urea (0.1 ml) was added to each sample. All samples were heat-treated at 50°C for 1 h and then subjected to centrifugation for 1 min at 10000 rpm. Approximately 5-15 µl of supernatant was filled into each electrophoretic well. Protein standards with precise molecular weights (markers) between 10 and 250 kDa were also charged in order to correctly determine the molecular weights of the proteins with unknown molecular weights. The run was conducted at 70 V for about 10 minutes in the concentration gel and at 120 V for about 2 hours in the separation gel. At the end of the electrophoretic run, the gel was immersed in Coomassie-based fixation solution (0.125% Coomassie Blue R 250, 40% methanol, 10% acetic acid) for 1 hour. Finally, the gel was immersed in a decolorizing solution (40% methanol, 10% acetic acid) overnight at 4°C and captured.

### Examples

### Example 1

### Process for the preparation of the collagen powder of the invention

The exact amount of crude collagen fibers, homogenized, is transferred to the container of a laboratory mixer, inside which a solution of AcOH (1.5%) in deionized water (at a ratio of 0.25 ml of AcOH per gram of collagen) is then added to obtain a collagen suspension with a weight-on-volume concentration of 3%_{wt/v}. The suspension is mixed for at least 1 hour at 50 rpm at room temperature. The resulting gel is transferred inside a second mixer and further mixed at 200 rpm, for 1 hour at 37°C. At this point, a proteolytic solution of pepsin (5% by weight of collagen) in AcOH (0.07%) is added to the mixing gel, and the system is kept under stirring (200 rpm) at 37°C for 4 hours. At the end of the enzymatic incubation, the collagen gel is dried by 'spray-drying'. Finally, the micrometric powder thus obtained is further dried by 'air-drying' for 12 hours at room temperature and stored for further processing.

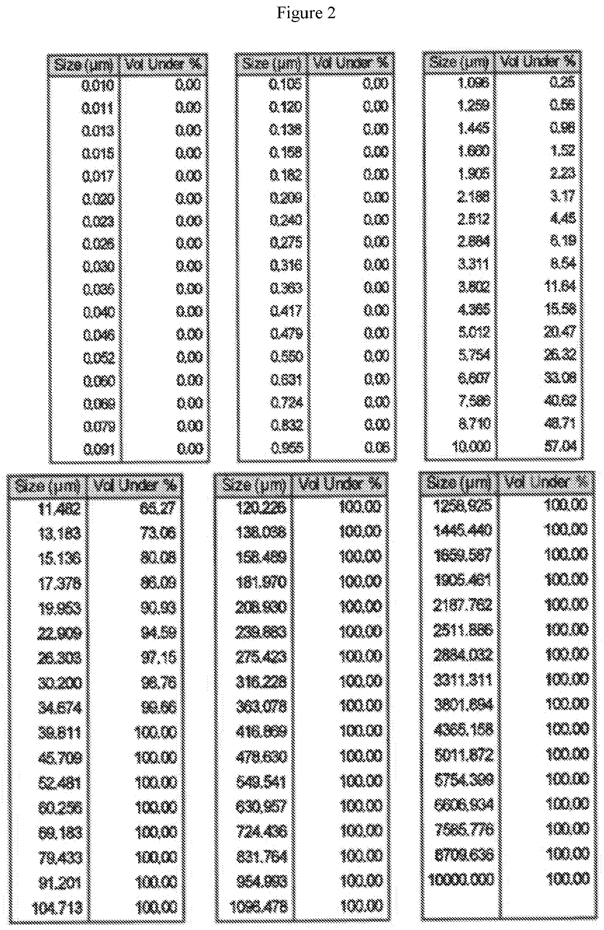

The powder so obtained shows the particle size of Figure 1, whose experimental data are set forth in the Table in Figure 2.

### Example 2

An injectable solution is prepared in pre-filled syringes comprising, for each 50 ml syringe, 80 mg of collagen powder from Example 1, water/saline solution, hyaluronic acid, chondroitin sulfate, buffers q.s. to pH.4.5

### Example 3

A medicated gauze comprising 20 mg of collagen powder from Example 1 is prepared, integrated in a 2%/gr/25cmq aqueous solution of glycerin.

### Example 4

A mixture of 100 mg of collagen powder from Example1, added with rice starch or cornstarch, a mixture of triglycerides, isopropanol and medical petroleum, is prepared to make a spray formulation - 10 g content in 100 ml spray cans.

### Example 5

A high-porosity medical silicone base is prepared, integrated with a thin layer of collagen - foil - charged with 100 mg of collagen powder from Example 1 for an area of 100 sq. cm.

### Example 6

A solution of product is prepared for vaginal irrigation in ampoules containing, for each 10 mL ampoule, 10 mg of collagen powder from Example 1, water/saline solution and, depending on the desired recommendations for use, one or more components selected from probiotics, postbiotics and prebiotics; a buffer solution q.s. to pH 4.5 is added to the resulting composition.

### Example 7

A solution of product is prepared in vaginal ovules containing 5 mg of Example 1 collagen powder, water, glycerol, lactic acid, and depending on the desired recommendation for use, one or more components selected from probiotics, postbiotics and prebiotics.

### Example 8

A vial is prepared with 80 mg of the powder from Example 1 to be used for the regeneration/bio-revitalization of dermis. The powder should be reconstituted with WFI before use.

### Example 9

A vial is prepared with 80 mg of the powder from Example 1 to be used for the visco-supplementation and regeneration in the synovia in cases of osteo-chondral damage. The powder should be reconstituted with WFI before use.

### EXPERIMENTAL TESTS

The collagen of the invention was subjected to several experimental tests.

The tested product is herein below referred to as "BIO ACTIVE COLL" which is collagen Type I powder, obtained by the process of Example 1.

### EXPERIMENTAL TEST 1

### CYTOTOXICITY BY DIRECT CONTACT TEST ON: "BIO ACTIVE COLL SUMMARY

A toxicological study was carried out on the test item "BIO ACTIVE COLL" aimed to evaluate any cytotoxic effect.

The following test was performed:
- cytotoxicity by direct contact according to ISO 10993-5:2009.

To perform the test, a subconfluent BALB/3T3 cell culture in exponential phase of growth was used.

The test item was applied to the monolayer of BALB/3T3 cells and was incubated at (37±1)°C in (5±1)% C0₂ atmosphere for 24 hours.

A qualitative evaluation was performed observing cell culture by means of an inverted microscope, while a quantitative evaluation was performed using the Neutral Red Uptake method (NRU).

The NRU is a method to measure cell viability using their capacity to incorporate and to bind a cellular viability dye, the Neutral Red.

### Qualitative evaluation

After incubation the cells were observed under the light microscope to evaluate the biological reactions.

After 24 hours of contact, in the cells treated with test sample no detectable zone around or under specimen has been observed (reactivity grade 0).

### Quantitative evaluation

After the qualitative evaluation cells were treated for 3 hours with the medium containing the cell viability dye and then with a Desorb Solution that allows to obtain a cell lysate. The optical density was then calculated after a 540 nm spectrophotometric reading.

Cells treated with test sample have shown a cell viability reduction of 3%.

On the basis of the results, interpreted according to ISO 10993-5:2009, the test item "BIO ACTIVE COLL" must be considered NOT CYTOTOXIC

### BIBLIOGRAPHY

- ISO 10993-5:2009 Biological evaluation of medical devices Part 5: Tests for in vitro cytotoxicity.

### EXPERIMENTATION

### Experimental Report

Eurofins Biolab is accredited by ACCREDIA ("Ente Italiano di Accreditamento") according to UNI CEI EN JSO/IEC 17025. The applied test method described in this document is included in the accredited test list (see http://www.eurofins.it/pharma/accreditamenti/).

### Characterisation

Mammal fibroblasts BALB/3T3 clone A31 (ATCC^{®} CCL163^{™}). Source: ATCC.

### Justification of assay system

Mammal fibroblasts BALB/3T3 were used for this test following recommendations of ISO 10993-5:2009 Annex A.

### Materials and equipment

Dulbecco's Modification of Eagle's Medium (DMEM) Dulbecco's Phosphate-Buffered Saline (DPBS) Penicillin-Streptomycin (Pen-Strep, P/S)
Foetal Bovine Serum (FBS) Water for injections (WFJ) Glacial acetic acid Neutral Red dye (NR) Ethanol (EtOH) Trypsin EDTA

### Trypan Blue

Common laboratory equipment Laminar flow filtered work area Biohazard Hood Inverted Microscope Diavert Microplate reader
C02 incubator Orbital shaker Chronometer Refrigerator
6 mm Whatman inert filter paper

### Controls

Dulbecco's Phosphate-Buffered Saline (DPBS, negative control)* Sodium Dodecyl Sulphate (SDS/SLS, positive control)*
*deposed on a 6 mm Whatman inert filter paper

| Media | DMEM 10% FBS |
|---|---|
| Routine culture medium: | 100 U/mL Penicillin |
| (Supplemented culture medium) | 100 U/mL Streptomycin |
| Stock Neutral Red solution (4 mglmL) | 0.4 g NR Dye 100 ml WFI |
| Neutral Red Medium (0.05 mglmL) | NR Stock in DMEM (1:80) |
| Ethanol/acetic acid solution: (NR Desorb Solution | Glacial Acetic acid (1 v/v%) |
| | Ethanol (50 v/v%) |
| | WFI (49 v/v%) |

### EXPERIMENTAL DESIGN

### PLATE PREPARATION

From a Mammal fibroblasts BAlB/3T3 culture, a suspension of 1 x 10⁵ cells/ml was prepared and dispensed in two 12-well plates subdivided in the following groups (triplicate)

| GROUPS PLATE 1 | | | | GROUPS PLATE 2 | | | |
|---|---|---|---|---|---|---|---|
| blank | vehicle | neg control | test sample | blank | vehicle | neg control | test sample |
| blank | vehicle | neg control | test sample | blank | vehicle | neg control | test sample |
| blank | vehicle | neg control | test sample | blank | vehicle | neg control | test sample |

- 1.2 ml of the cell suspension (1.2 x 10⁵ cells/well) was pipetted in the Vehicle, Negative/Positive controls and Test Sample wells.
- 1.2 ml of supplemented culture medium alone (without cells) was pipetted in Blank wells.

The two plates were incubated at (37±1)°C in a (5±1)% C02 atmosphere, allowing cells sedimentation and the constitution of a subconfluent monolayer.

### TREATMENT

After 24 hours from cells seeding, the plates were observed to verify that a subconfluent (approximately 80% confluence) monolayer was present.

Supplemented culture medium was replaced with fresh one 7 1.2 ml/ well.

Blank, Vehicle, Negative/Positive controls and Test Sample wells were treated as follows:

### Blank

Supplemented culture medium alone (without cells). [6 replicates]

### Vehicle

Supplemented culture medium with an inert filter paper placed in the middle of each well. [6 replicates]

### Test sample preparation

As Requested by the Sponsor, the powder contained in the vial (80 mg) was suspended with 2 ml ofWFI to reach the concentration of 40 mg/ml.

50 µL of the test sample were deposed on inert filter paper placed then in the middle of each well. [3 replicates]

### Negative control preparation

The negative control was represented by 50 µL of DPBS deposed on an inert filter paper placed in the middle of each well. [3 replicates]

### Positive control preparation

The positive control was represented by 50 µL of 0.4% solution of SOS /SLS deposed on an inert filter paper placed in the middle of each well. [3 replicates]
The plates were then incubated in a thermostat at (37±1)°C in a (5±1)% C02 atmosphere for 24 hours.

After this contact time, the plates were observed under an inverted microscope and biological reactions were evaluated following a O to 4 scale according to ISO 10993-5:2009. Each well was emptied, washed with DPBS and treated with 1200 µL of Neutral Red Medium for 3 hours at (37±1)°C in a (5±1)% CO₂ atmosphere. Thereafter each well was washed with DPBS, totally dried and then treated with 1800 µL of NR Desorb Solution. The plate was put in stirring for at least 15 minutes to homogenize the solution.

The absorbance of the resulting solution was measured at 540 nm in a microtiter plate reader

### OBSERVATIONS

### Qualitative evaluation

The biological reactivity (cell degeneration and malformations) was evaluated after 24 hours of incubation with a scale ranging from 0 to 4, according to ISO 10993-5 as shown in the following table:

| GRADE | REACTIVITY | DESCRIPTION OF REACTIVITY ZONE |
|---|---|---|
| 0 | None | No detectable zone around or under specimen |
| 1 | Slight | Some malformed or degenerated cells under specimen |
| 2 | Mild | Zone limited to area under specimen |
| 3 | Moderate | Zone extending specimen size up to 1.0 cm |
| 4 | Severe | Zone extending farther than 1.0 cm beyond specimen |

### Quantitative evaluation

Optical density was measured at 540 nm (Gen5 - Biotek). Percentages of cell viability was calculated according to the formula: $% of cellular viability = \frac{{OD}_{test product} - {OD}_{blank}}{{OD}_{vehicle} - {OD}_{blank}} \times 100$

### INTERPRETATION OF RESULTS

### Qualitative evaluation

The achievement of a numerical grade greater than 2 was considered a cytotoxic effect.

### Quantitative evaluation

A cellular viability reduction >30% was considered a cytotoxic effect.

### ACCEPTABILITY CRITERIA

### Qualitative evaluation

Vehicle control = 0
Negative control = 0
Positive control ≥ 3

### Quantitative evaluation

The mean OD of vehicle must be ≥ 0.3
The negative control % cellular viability must be ≥ 90% The positive control % cellular viability must be < 70% Coefficient of Variation % of each group must be ≤ 15%

### CONCLUSIONS

The results, interpreted according to ISO 10993-5:2009, showed that the test item "BIO ACTIVE COLL" must be considered NOT CYTOTOXIC.

### EXPERIMENTAL TEST 2

### DELAYED HYPERSENSITIVITY TEST (GPMT) ON "BIO ACTIVE COLL SUMMARY

On the test item "BIO ACTIVE COLL" was carried out a biological evaluation aimed at identifying its potential sensitising effects by means of following test:
- Delayed hypersensitivity test (GPMT test) according to ISO 10993-10:2021.

In the Guinea Pig Maximisation Test 15 guinea pigs were used for the main test, 10 of which were treated with the test item and 5 were used as a control group and 3 for preliminary test.

The maximization test consists of a preliminary test, an induction phase and a challenge phase.

### Preliminary test

A preliminary test was intended to determine the concentration of the test sample to be used in the main test.

For the topical induction phase, the highest concentration that causes mild to moderate erythema but does not otherwise adversely affect the animal was selected. For the challenge phase, the highest concentration that produces no erythema was selected.

To select the sample dilution, four occlusive patches with 0.5 ml of the undiluted sample and diluted sample (90%, 80% and 70% in Sodium Chloride Injection) were applied to the dorsum of three additional animals. The dressing was left in place for 24 hours.

### Main test

### Induction phase

Guinea pigs were treated with 3 pairs of intradermal injections (each dose of 0.1 ml) thus subdivided:
1^{st} stable emulsion of Freund's complete adjuvant (FCA) in Sodium Chloride Injection 50:50 (v:v);
2^{nd} test sample for treated animals, Sodium Chloride Injection for control animals;
3^{rd} test sample diluted 50:50 (v:v) with stable emulsion of FCA and Sodium Chloride Injection (50%) for treated animals, Sodium Chloride Injection diluted 50:50 (v:v) with stable emulsion of FCA and Sodium Chloride Injection (50%) for control animals.

6 days after performing the intradermal injections- treated and control ones - a local application was performed on all the animals by massaging 0.5 ml of Sodium Lauryl Sulfate at 10% in vaseline.

7 days after performing the intradermal injections, 0.5 ml/animal of test sample was applied to the skin in 10 treated animals for 48 hours.

The same treatment was performed on control group using Sodium Chloride Injection.

### Challenge

13 days after topical induction phase on all the animals, both treated and control ones, the challenge phase was carried out by applying 0.5 ml of test sample on the right flank and 0.5 ml of Sodium Chloride Injection on the left side. Bandaging was left for 24 hours.

48 and 72 hours after starting the challenge phase, the reactions of both treated and control animals were evaluated.

No abnormalities were observed in treated animals with the test sample. No abnormalities were observed in control animals.

On the basis of the results, interpreted according to ISO 10993-10, the test item "BIO ACTIVE COLL" must be considered NOT SENSITIZING.

### INTRODUCTION

This study has been carried out at the Test Facility Eurofins Biolab S.r.J. on behalf of the Sponsor on the test item "BIO ACTIVE COLL"

### BIBLIOGRAPHY

- ISO 10993-10:2021 Biological evaluation of medical devices Part 10: Tests for skin sensitization
- ISO 10993-2: 2006 Biological evaluation of medical devices: Part 2: Animal welfare requirements

### TEST METHOD

| Characterization | |
|---|---|
| Species: | Albino guinea pigs |
| Strain : | Dunkin-Hartley |
| N.: | 15+3 |
| Weight: | According to ISO 10993-10 par 6.5.3, at the beginning of the test (300 - 500 g) |
| Age: | Young Adult |
| Sex: | Male |
| Supplier: | Envigo - Bresso (Ml) - Italy |
| Receiving number: | 034 |
| Food | 8GP22 Batch: 380001 (from 07/03/2022 to 27/03/ 2022) |
| | 8GP22 Batch: 383201 (from 28/03/2022 to 31/03/2022) |

### Justification of the assay system

Guinea Pig has been used for this test because of the recommendation of ISO 10993-10 - current edition.

### Quarantine

Before allocation to the study, the animals were kept in quarantine according to internal procedure. During this period, they were daily observed by a veterinarian. At the end of the quarantine period, the animals were carefully examined in order to evaluate their suitability for the study.

### Animal selection

Animals used in the study were randomly selected among the ones available at the time of the study.

### Caging

The animals were caged according to internal procedure.

The housing room was lighted with fluorescent lamps and maintained with cycles of 12 hours of light and 12 hours of dark. Room temperature and humidity were regulated by a conditioning plant and were daily monitored. Continuous recordings of the housing conditions are being retained in Eurofins Biolab S.r.l. files.

### Cleaning and disinfection

The cages and the housing room were cleaned before animal accommodation, then periodically cleaned and disinfected.

### Feeding

Animals have been fed with standard pellet complete diet supplied by the authorized breeder.

### Watering

Filtered tap water from local network was supplied ad libitum

### Animals' identification

The animals were identified with an indelible colouring in different areas of the body as:

### Animals' identification

The animals were identified with an indelible colouring in different areas of the body as:

| | | |
|---|---|---|
| Tail | (C) | 3 |
| Head-tail | (TC) | 4 |
| Right forepaw | (ZAD) | 5 |
| Left forepaw | (ZAS) | 6 |
| Right hind leg | (ZPD) | 7 |
| Left hind leg | (ZPS) | 8 |
| Abdomen | (P) | 9 |
| Head-abdomen | (TP) | 10 |

### PRELIMINARY TEST

A preliminary test was intended to determine the concentration of the test sample to be used in the main test.

For the topical induction phase, the highest concentration that causes mild to moderate erythema but does not otherwise adversely affect the animal was selected. For the challenge phase, the highest concentration that produces no erythema was selected. To select the sample dilution, four occlusive patches with 0.5 ml of the undiluted sample and diluted sample (90%, 80% and 70% in Sodium Chloride Injection) were applied to the dorsum of three additional animals; the fur was removed 24 hours before the treatment.

The dressings were left in place for 24 hours.

### Results:

| **ANIMAL N.** | **24 HOURS AFTER PATCH REMOVAL** | | | |
|---|---|---|---|---|
| | **Neat (100%)** | **Diluted 90%** | **Diluted 80%** | **Diluted 70%** |
| 1 | 0 | 0 | 0 | 0 |
| 2 | 0 | 0 | 0 | 0 |
| 3 | 0 | 0 | 0 | 0 |

| | | | | |
|---|---|---|---|---|
| 0 =No symptoms | | | | |

After 24 hours of bandage removal, no erythema was observed in any treated sites.

### SAMPLE PREPARATION

The powder contained in the vial (80mg) was suspended in 2ml of W FI (Batch: 1902401) to reach the concentration of 40 mg/ml before use.

### EXPERIMENTAL DESIGN

Experimental design consisted of one group of 10 treated animals (group 1) and one group of 5 control animals (group 2).

The animals were allocated into groups as follows:

| GROUP | INDUCTION | | CHALLENGE REACTION |
|---|---|---|---|
| | lntradermal injection | Topical application | Challenge |
| 1 | 1 Stable emulsion of Freund's complete adjuvant (FCA) in Sodium Chloride Injection 50:50 (v:v) | Test sample | Test sample |
| | 2 test sample | | |
| | 3 test sample diluted 50:50 (v:v) with stable emulsion of FCA and Sodium Chloride Injection (50%) | | |
| 2 | 1 Stable emulsion of Freund's complete adjuvant (FCA) in Sodium Chloride Injection 50:50 (v:v) | Sodium Chloride Injection | Test sample |
| | 2 Sodium Chloride Injection | | |
| | 3 Sodium Chloride Injection diluted 50:50 (v:v) with stable emulsion of FCA and Sodium Chloride Injection (50%) | | |

| | | | |
|---|---|---|---|
| At maximum 10 animals for each cage; cages have been identified via a tag | | | |

### TREATMENT

The main test consisted of an induction phase and a challenge phase.

### Skin preparation

About 7 hours before intradermal injection and about 23 hours before topical application of induction phase, fur was removed by shaving a 50 cm² wide area on the interscapular region of the animals.

About 23 hours before the challenge phase the fur on both flanks of animals was removed.

### Administration

### Induction phase

### Day 0 - treated and control groups

Three pairs of 0.1 ml intradermal injections were made in the interscapular region of each animal, on each side of the midline, according to the experimental design.

### Day 6- treated group and control group

6 days after the beginning of treatment a topical application on the all animals was made, with slight massage of 0.5 ml of Sodium Lauryl Sulfate 10% in vaseline.

### Day 7 - treated and control group

7 days after the intradermal injections, 0.5 ml of the test sample were applied to each animal and held in place with an occlusive patch. The application was made so as to cover the intradermal injection sites.

The dressing was left in place for 48 hours.

The same treatment was performed on the control group, using Sodium Chloride Injection instead of the test substance.

### Challenge

### Day 13 after topical induction phase - treated group and control group

An occlusive patch with 0.5 ml of the assay sample was applied to the right flank of all 15 guinea pigs, while Sodium Chloride Injection was applied on the left side. The dressing was left in place for 24 hours.

### OBSERVATIONS

24 ± 2 and 48 ± 2 hours after removal of the patches, all treated and control animals were evaluated for a skin reaction.

The intensity of erythema and/or oedema was evaluated according to the following scale (Magnusson and Kligman -18010993-10):

| Patch test reaction | Grading scale |
|---|---|
| No visible change | 0 |
| Discrete or patchy erythema | 1 |
| Moderate and confluent erythema | 2 |
| Intense erythema and swelling | 3 |

### INTERPRETATION OF RESULTS

Generally, Magnusson and Kligman grades of 1 or greater in the test group indicate sensitizatio, provided grades of less than 1 are seen in control animals. If grades of 1 or greater are noticed in control animals, the reactions of test animals which exceed the most severe reaction in control animals are presumed to be due to sensitization.

If the response is equivocal, re-challenge is recommended to confirm the results from the first challenge. The outcome of the test is presented as the frequency of positive challenge results in the treated and control animals.

### RESULTS

### SKIN REACTIONS IN TREATED ANIMALS AFTER REMOVAL OF THE PATCH OF CHALLENGEPHASE

| ANIMAL N. | TIME AFTER REMOVAL OF THE PATCH | |
|---|---|---|
| | 24 hours | 48 hours |
| 1 | 0 | 0 |
| 2 | 0 | 0 |
| 3 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 10 | 0 | 0 |

No abnormalities were observed in animals treated with the test sample.

### SKIN REACTIONS IN CONTROL ANIMALS AFTER REMOVAL OF THE PATCH OF CHALLENGE PHASE

| ANIMAL N. | TIME AFTER REMOVAL OF THE PATCH | |
|---|---|---|
| | 24 hours | 48 hours |
| 1 | 0 | 0 |
| 2 | 0 | 0 |
| 3 | 0 | 0 |
| 4 | 0 | 0 |
| 5 | 0 | 0 |

| | | |
|---|---|---|
| No abnormalities were observed in control animals. % sensitising treated guinea pigs: 0% | | |

### CONCLUSIONS

On the basis of the results, interpreted according to ISO 10993-10, the test item "BIO ACTIVE COLL" must be considered NOT SENSITIZING.

### EXPERIMENTAL TEST 3

### ACUTE SYSTEMIC TOXICITY TEST ON BIO ACTIVE COLL

### SUMMARY

On the test item "BIO ACTIVE COLL" a biological evaluation was carried out to evaluate its toxicity by means of the following test:
- Systemic toxicity test according to ISO 10993-11:2017

To perform the systemic toxicity test, the test sample was intraperitoneally injected in ratio 50 ml/Kg of animal weight in one group of 5 mice.

Sodium Chloride Injection was used as control and injected with the same modality in another group of 5 animals.

The animals were observed immediately after the injection and after 4, 24, 48 and 72 hours. Clinical signs, systemic effects and mortality were recorded.

Body weight has been measured immediately before the injections, 24, 48 and 72 hours after the injections.

### Clinical symptoms

In none of the treated and control animals' toxic signs or symptoms were observed.

### Mortality

In none of the treated and control animals' mortality was observed.

### Weight increase

No weight loss was recorded in any treated and control animal.

On the basis of the results, interpreted according to ISO 10993-11:2017, the test item "BIO ACTIVE COLL" DOESN'T CAUSE toxic symptoms and SATISFIES the requirements of the test.

### TEST METHOD

| | |
|---|---|
| Species: | Mouse |
| Strain: | ICR (CD-1^{®}) |
| N.: | 10 (5 + 5) |
| Sex: | Male |
| Weight: | 17.2 - 19.5 g at the beginning of the test |
| Age: | Young adult |
| Supplier : | Charles River - Calco (LC) - Italy |
| Receiving number: | 033 |
| Food: | 4RF1 Batch: 382703 |
| Bedding | PF3903 Batch: 70965 |

### Justification of assay system

Mouse has been used for this test because of the recommendation of ISO 10993-11-current edition.

### Caging

The animals were caged according to internal procedure.

The housing room was lit with fluorescent lamps 12 hours per day. Room temperature and humidity were regulated by a conditioning plan and were monitored continuously. Recordings of the housing conditions are being retained in Eurofins Biolab S.r.l. files.

### Cleaning and disinfection

The cages and the housing room were cleaned before animal accommodation, then periodically cleaned and disinfected.

### Feeding

Animals have been fed with standard pellet complete diet supplied by the authorized breeder.

### Watering

Filtered tap water from local network was supplied ad libitum.

### Animal identification

Each animal was identified with an indelible painting in different parts of the body:

| | |
|---|---|
| No sign | 1 |
| Head | 2 |
| Back | 3 |
| Tail | 4 |
| Head-Tail | 5 |

Cages were identified by a tag.

### Quarantine

Before being used in this study, the animals were kept in quarantine according to internal procedure. During this period, they were daily observed.

At the end of the quarantine period the animals were carefully examined in order to evaluate their suitability for the study.

### Animal selection

The animals used for this study were randomly selected from those suitable and available at that time.

### EXPERIMENTAL DESIGN

The experimental design consisted of 2 groups (1 treated and 1 control) each consisting of five male mice. The animals were subdivided in groups as follow:

| GROUP | ADMINISTRATION LIQUID | INJECTION ROUTE | DOSE (ml/kg) | N. ANIMALS |
|---|---|---|---|---|
| Treated | Test Sample | I.P. | 50 | 5 |
| Control | Sodium Chloride Injection | I.P. | 50 | 5 |

| | | | | |
|---|---|---|---|---|
| I.P.: intra-peritoneal | | | | |

### Sample preparation

As Requested by the Sponsor, the powder contained in the vial (80mg) was suspended with 2ml of WFI to reach the concentration of 40 mg/ml before use.

### Treatment

Animals have been treated with a single injection of test sample (treated groups) and Sodium Chloride Injection (control groups) with a dose of 50 ml/kg.

### Body weight measurement

Body weight of all animals has been measured immediately before the injections, 24, 48 and 72 hours after the injections.

### OBSERVATIONS

All animals have been observed after injection and after 4, 24, 48 and 72 hours.

Eventual clinical signs (time of onset, degree and duration) and/or eventual mortality have been recorded for each animal.

Animals were observed for the following systemic effects: tremors, hair bristling, diarrhoea, abdominal pain, sialorrhoea, depression state of sensorium, state of excitement, polypnoea, hypopnoea, tachycardia, cyanosis, ataxia, convulsions, nose-bleeding.

Other clinical signs described in ISO 10993-11, Annex C, (if present) has been recorded.

### INTERPRETATION OF THE RESULTS

The test conditions are satisfied if none of the animals treated with the sample show significantly greater biological reactivity than control group.

If any of the animals treated with the sample show slight signs of biological reactivity, and no more than one animal show gross symptoms of biological reactivity or die, the test must be repeated using groups of 10 mice.

The conditions of the repeated test are satisfied if during observation period none of the animals treated with the sample show a biological reactivity greater than the animals treated with the control.

If two or more mice die, if two or more mice show abnormal symptoms as convulsions or weakness or if the weight loss is greater than 10% in 3 or more animals the test substance does not satisfy the requirements of the test.

### RESULTS

### WEIGHT AND CLINICAL SYMPTOMS OF ANIMALS TREATED W ITH 50

ml/k:Q OF TEST SAMPLE

| ANIMAL N. | START WEIGHT (Q) | END WEIGHT (g) | CLINICAL SYMPTOMS (h) | | | | |
|---|---|---|---|---|---|---|---|
| | | | 0 | 4 | 24 | 48 | 72 |
| 1 | 17.2 | 21.0 | 0 | 0 | 0 | 0 | 0 |
| 2 | 18.4 | 22.2 | 0 | 0 | 0 | 0 | 0 |
| 3 | 17.2 | 20.3 | 0 | 0 | 0 | 0 | 0 |
| 4 | 17.3 | 21.0 | 0 | 0 | 0 | 0 | 0 |
| 5 | 17.4 | 21.7 | 0 | 0 | 0 | 0 | 0 |

| | | | | | | | |
|---|---|---|---|---|---|---|---|
| 0: No Symptoms, 1:tremors, 2:hair bristing, 3:diarrhoea, 4:abdommal pam, 5:shalorrhoea, 6:depress,on state of sensorium, 7:state of excitement, 8:polypnoea, 9:hypopnoea, 10:tachycardia, 11:cyanosis, 12:ataxia, 13:convulsions, 14:nose-bleeding, M:death. | | | | | | | |

### WEIGHT AND CLINICAL SYMPTOMS OF ANIMALS TREATED WITH 50 ml/kg OF COTTON SEED OIL

| ANIMAL N. | START WEIGHT (Q) | END WEIGHT (g) | CLINICAL SYMPTOMS (h) | | | | |
|---|---|---|---|---|---|---|---|
| | | | 0 | 4 | 24 | 48 | 72 |
| 1 | 18.0 | 21.5 | 0 | 0 | 0 | 0 | 0 |
| 2 | 18.1 | 20.7 | 0 | 0 | 0 | 0 | 0 |
| 3 | 17.2 | 19.8 | 0 | 0 | 0 | 0 | 0 |
| 4 | 17.7 | 22.2 | 0 | 0 | 0 | 0 | 0 |
| 5 | 19.5 | 21.9 | 0 | 0 | 0 | 0 | 0 |

| | | | | | | | |
|---|---|---|---|---|---|---|---|
| 0: No Symptoms, 1:tremors, 2:hair bristling, 3:diarrhoea, 4:abdommal pam, 5:shalorrhoea, 6:depress,on state of sensorium, 7:state of excitement, 8:polypnoea, 9:hypopnoea, 10:tachycardia, 11:cyanosis, 12:ataxia, 13:convulsions, 14:nose-bleeding, M:death. | | | | | | | |

### DISCUSSION OF RESULTS

### Weight increase

No weight loss was recorded in any treated and control animal.

### Mortality

In none of the treated and control animals' mortality was observed.

### Clinical symptoms

In none of the treated and control animals' toxic signs or symptoms were observed.

### CONCLUSIONS

On the basis of the results, interpreted according to ISO 10993-11:2017, the test item "BIO ACTIVE COLL" doesn't cause toxic symptoms and satisfies the requirements of the test.

### EXPERIMENTAL TEST 4

### RATIONALE ON MATERIAL-MEDIATED PYROGENICITY FOR BIO ACTIVE COLL

### Scope

The aim of this document is to provide a rationale on the material-mediated pyrogenicity endpoint for the device Bio Active Coll.

### Introduction

Pyrogenicity is among the biological endpoints suggested to be evaluated in the contest of the biological evaluation of a medical device according to ISO 10993-1:2018.

ISO 10993-11:2017 describe the approach to be used for the evaluation of pyrogenicity of medical devices and states that "Pyrogenicity is the ability of a chemical agent or other substance to produce a febrile response. Pyrogenic responses may be material-mediated, endotoxin-mediated, or mediated by other substances, such as components of gram-positive bacteria and fungi".

As defined by ISO 10993-11:2017, for detection of material-mediated pyrogenicity on medical devices, the rabbit pyrogen test is recommended. The test shall be performed according to the method described in the United States Pharmacopoeia, the European Pharmacopoeia and the Japanese Pharmacopoeia.

### Device description

Based on information provided by the Sponsor, "Bio Active Coll - TYPE1 PROFIL" is intended to be used for the regeneration of the dermic and sub-dermic cutaneous tissue. The device is intended to be applied topically on the skin.

The device consists in a water-soluble sterile powder made of collagen (Type I) from equine origin. At the time of use, the device shall be dissolved in water for injection or saline solution, at the concentration of 20 40mg/ml.

The device is supplied in vials containing 80 mg of powder

### Assessment of material mediated pyrogenicity for "Bio Active Coll"

According to USP <151>, the test should be performed using an extract of the device, which should be injected into an ear vein of 3 rabbits.

This test has not been performed on the device nor has been planned given its characteristics (substance-based mixture based on equine-origin collagen) in order to avoid any pain, suffering, distress or lasting harm to the animals as required by ISO 10993-2:2006.

In fact, due to the physical nature of the medical device under evaluation, the required intravenous injection in rabbit (according to ISO 10993-11 and USP <151>) has a high probability of resulting in the animal death and animal suffering without producing relevant data to be used to characterize the potential risk of pyrogenicity related to constituents of the device. Therefore, for animal welfare, it is highly inadvisable and discouraged to perform the material-mediated pyrogenicity test with the medical device, as such.

An alternative would be to furtherly dilute the sample to obtain a test solution suitable to be intravenously injected. However, this approach results in obtaining a test solution not representative of the medical device in contact with the patient. Therefore, test results are not useful to characterize the potential risk of material-mediated pyrogenicity of the medical device.

In addition, Annex G of ISO 10993-11:2017, states:
"It is not necessary to test all new medical devices for in vivo pyrogenicity. However, materials containing substances that have previously elicited a pyrogenic response, and/or new chemical entities where the pyrogenic potential is unknown should be evaluated for material-mediated pyrogenicity".

In addition, collagen is not listed by the Annex G of ISO 10993-11:2017 among the substances which are known to generate a pyrogenic response, therefore a material-mediated pyrogenic response is not expected for collagen.

### Conclusions

Based on the above observations and since according to ISO 10993-2 "Animal tests are deemed to be justified only when they have been shown to be relevant and reliable for the purposes for which they are undertaken", in vivo rabbit pyrogen test for material-mediate pyrogenicity is deemed not applicable for the device under evaluation "Bio Active Coll".

### EXPERMENTAL TEST 5

### IN VITRO MAMMALIAN CELL GENE MUTATION ASSAY (THYMIDINE KINASE LOCUS/TK+/-) IN L5178Y MOUSE LYMPHOMA CELLS WITH BIO ACTIVE COLL

### Summary

The test item BIO ACTIVE COLL was assessed for its potential to induce mutations at the mouse lymphoma thymidine kinase locus (tk+/-) using the L5178Y cell line.

For the short-term experiments (STE), the solved test item was incubated with cells for 4 hours. The STEs were performed independently: with (STE(+)) and without (STE(-)) metabolic activation. For the long-term experiment (LTE), the solved test item was incubated with cells for 24 hours. The LTE was performed without metabolic activation.

The solved test item was investigated at the following concentrations:
STE(-): 0.25, 0.50, 1, 3 and 5 mg/mL
STE(+): 0.25, 0.50, 1, 3 and 5 mg/mL
LTE: 0.25, 0.50, 1, 3 and 5 mg/mL

No precipitation of the solved test item was noted in the experiments. No growth inhibition was observed in STE(-).

Growth inhibition was observed in STE(+). The relative total growth (RTG) was 69%, for the highest concentration (5 mg/mL) evaluated.

Also, growth inhibition was observed in LTE. The relative total growth (RTG) was 26% for highest concentration (5 mg/mL) evaluated.

No biologically relevant increase of mutants was found in any experiment. The global evaluation factor (GEF) was not exceeded by the induced mutant frequency.

EMS, MMS, and B[a]P were used as positive controls and demonstrated distinct and biologically relevant responses in mutation frequency. Additionally, MMS and B[a]P significantly increased the number of small colonies, thus proving the efficiency of the test system to indicate potentially clastogenic effects.

### Summary Conclusion

In conclusion, in this in vitro Mammalian Cell Gene Mutation Assay (Thymidine Kinase Locus/tk+/-) in L5178Y Mouse Lymphoma Cells, under the experimental conditions reported, the test item BIO ACTIVE COLL is considered to be non-mutagenic.

**Table 1: STE(-) - Summary**

| **Test Group** | **Conc. [mg/mL]** | **RCE^{a} [%]** | **RTG^{b} [%]** | **MF^{c} [mutants/10⁶ cells]** | **IMF^{d} (mutants/10⁶ cells]** | **GEF^{e} exceeded** |
|---|---|---|---|---|---|---|
| NC | 0 | 89 | 84 | 151 | / | / |
| | 0 | 109 | 103 | | / | / |
| SC | 0 | 100 | 100 | 150 | / | / |
| | 0 | | | | / | / |
| Test Item | 0.25 | 112 | 90 | 132 | -18 | - |
| | 0.5 | 114 | 116 | 159 | 9 | - |
| | 1 | 81 | 85 | 174 | 24 | - |
| | 3 | 90 | 84 | 173 | 23 | - |
| | 5 | 81 | 78 | 191 | 40 | - |
| EMS | 300 µg/mL | 60 | 49 | 1524 | 1373 | + |
| MMS | 10 µg/mL | 85 | 64 | 816 | 665 | + |

| | |
|---|---|
| NC: | Negative control |
| SC: | Solvent control (Water (10%)) |
| a: | Relative cloning efficiency, RCE = [(CE _{test group} / CE _{of correspondin controls}) x 100], Cloning efficiency, CE = ((-ln (((96 - (mean Plate 1, Plate 2)) / 96)) / 1.6) x 100) |
| b: | Relative total growth, RTG = (RSG x RCE) / 100 |
| c: | Mutant frequency, MF = {-ln (negative cultures/total wells (selective medium)] / -ln [negative cultures/total wells (non-selective medium)]}×800 |
| d: | induced mutant frequency, IMF = mutant frequency sample - mean value mutant frequency corresponding controls |
| e: | Global evaluation factor, GEF (126 mutants/10⁶ cells); +: GEF exceeded, -: GEF not exceeded |
| EMS: | Ethyl methanesulfonate |
| MMS: | Methyl methanesulfonate |

**Table 2: STE⁽⁺⁾ - Summary**

| **Test Group** | **Conc. [mg/mL]** | **RCE^{a} [%]** | **RTG^{b} [%]** | **MF^{c} [mutants/10⁶ cells]** | **IMF^{d} [mutants/10⁶ cells]** | **GEF^{e} exceeded** |
|---|---|---|---|---|---|---|
| NC | 0 | 102 | 106 | 128 | / | / |
| | 0 | 99 | 105 | | / | / |
| SC | 0 | 100 | 100 | 123 | / | / |
| | 0 | | | | / | - |
| Test Item | 0.25 | 95 | 92 | 168 | 45 | - |
| | 0.5 | 113 | 104 | 122 | -1 | - |
| | 1 | 110 | 127 | 155 | 32 | - |
| | 3 | 115 | 115 | 112 | -11 | - |
| | 5 | 64 | 69 | 233 | 110 | - |
| B[a]P | 3.5 µg/mL | 71 | 39 | 791 | 668 | + |

| | |
|---|---|
| NC: | Negative control |
| SC: | Solvent control (Water (10%)) |
| a: | Relative cloning efficiency, RCE = [(CE .. group / CE _{of corresponding controls}) x 100], Cloning efficiency, CE = ((-ln (((96 - (mean Plate 1, Plate 2)) / 96)) / 1.6) x 100) |
| b: | Relative total growth, RTG = (RSG x RCE) / 100 |
| c: | Mutant frequency, MF = {-ln (negative cultures/total wells (selective medium)] / -ln [negative cultures/total wells (non-selective medium)]}×800 |
| d: | Induced mutant frequency, IMF = mutant frequency sample - mean value mutant frequency corresponding controls |
| e: | Global evaluation factor, GEF (126 mutants/10⁶ cells); +: GEF exceeded, -: GEF not exceeded |
| BlaIP: | Benzolalpyrene |

**Table 3: LTE - Summary**

| **Test Group** | **Conc. [mg/mL]** | **RCE^{a} [%]** | **RTG^{b} [%]** | **MF (mutants/10⁶ cells)** | **IMF^{d} [mutants/10⁶ cells)** | **GEF^{e} exceeded** |
|---|---|---|---|---|---|---|
| NC | 0 | 84 | 79 | 71 | / | / |
| | 0 | 101 | 113 | | / | / |
| SC | 0 | 100 | 100 | 58 | / | / |
| | 0 | | | | / | - |
| Test Item | 0.25 | 87 | 88 | 95 | 37 | - |
| | 0.5 | 112 | 105 | 49 | -9 | - |
| | 1 | 101 | 93 | 70 | 13 | - |
| | 3 | 103 | 35 | 71 | 13 | - |
| | 5 | 86 | 26 | 112 | 54 | - |
| EMS | 200 µg/mL | 56 | 28 | 2418 | 2361 | + |
| MMS | 10 µg/mL | 60 | 48 | 657 | 599 | + |

| | |
|---|---|
| NC: | Negative control |
| SC: | Solvent control (Water (10%)) |
| a: | Relative cloning efficiency, RCE = [(CE _{test group} / CE _{of corresponding controls}) x 100], Cloning efficiency, CE = ((-ln (((96 - (mean Plate 1, Plate 2)) / 96)) / 1.6) x 100) |
| b: | Relative total growth, RTG = (RSG x RCE) / 100 |
| c: | Mutant frequency, MF = {-ln [negative cultures/total wells (selective medium)] / -ln [negative cultures/total wells (non-selective medium)]}×800 |
| d: | Induced mutant frequency, IMF = mutant frequency sample - mean value mutant frequency corresponding controls |
| e: | Global evaluation factor, GEF (126 mutants/10⁶ cells); +: GEF exceeded, -: GEF not exceeded |
| EMS: | Ethyl methanesulfonate |
| MMS: | Methyl methanesulfonate |

### Aim of the Study

Mammalian cell culture systems are used to detect mutations induced by chemical substances. This in vitro experiment is conducted to assess the potential of the test item to induce gene mutations by means of a thymidine kinase (tk) assay using the mouse lymphoma cell line L5178Y. The thymidine kinase assay detects base pair mutations, frameshift mutations, small, large and non-lethal deletions and rearrangements of the relevant chromosomes.

The kinase catalyses the reaction of thymidine and ATP to form TMP (thymidine 5'-mono-phosphate) and ADP. However, it also phosphorylates the pyridine analogue triflurothymidine (TFT) to its cytostatic and cytotoxic trifluoro-thymidine-monophosphate derivative. By inactivating the functional allele of tk+/-, cells are resistant to TFT. Thus, mutant cells (tk-/-) are capable of proliferation in the presence of TFT, whereas normal cells (tk+/-) are not.

Cells in suspension are exposed to the test chemical, both with and without an exogenous source of metabolic activation (MA), for a suitable period of time, and then sub-cultured to determine cytotoxicity and to allow phenotypic expression prior to mutant selection.

Cytotoxicity is determined by relative total growth (RTG). The treated cultures are maintained in growth medium for a sufficient period of time, to allow near-optimal phenotypic expression of induced mutations.

Following phenotypic expression, mutant frequency (MF) is determined by seeding known numbers of cells in medium containing the selective agent to detect mutant colonies, and in medium without selective agent to determine the cloning efficiency. After a suitable incubation time, colonies are counted. Mutant frequency is calculated based on the number of mutant colonies corrected by the cloning efficiency at the time of mutant selection.

### Justification for the selection of the Test System

OECD (2016), Test No. 490 (In Vitro Mammalian Cell Gene Mutation Tests Using the Thymidine Kinase Gene) and ISO/TR 10993-33:2015 (Guidance on tests to evaluate genotoxicity - Supplement to ISO 10993-3) recommend using the cell line L5178Y

### Characterisation of the Test Item

The identity of the test item was inspected upon delivery at the test facility (e.g. test item name, batch no., and additional data were compared with the label) based on the following specifications provided by the sponsor.

The following listed information applies to the sample as received.

| | |
|---|---|
| Name: | BIO ACTIVE COLL |
| Batch No.: | 03/21 |
| Sterility: | yes |
| Sterilisation Procedure: | γ-Irradiation |
| Type of Material: | Natural Polymer |
| Storage Conditions: | Room temperature, cool dry place |
| Expiry Date: | not applicable |
| Safety Precautions: | The routine hygienic procedures were sufficient to assure personnel health and safety. |

### Preparation

The preparation was carried out in compliance to ISO 10993-3: 2014 "Tests for genotoxicity, carcinogenicity and reproductive toxicity":
- Can the test sample be dissolved/suspended in an appropriate solvent compatible with the test system?
   Yes: Use method A
   No: Determine the percentage of extractables in the test sample (pre-test method B - A.3.3.4).
- Is the percentage of extractables ≥ 0.5% for devices ≥ 0.5 g or ≥ 1% for devices < 0.5 g?
- Yes: Use methodB
   No: Use method C

The solvent was compatible with the survival of the cells and the S9 activity.

### Controls

Negative or solvent as well as positive controls are included in each experiment.

### Negative Control

Negative controls (RPMI) are treated the same way as all test groups.

### Solvent Control

| | |
|---|---|
| Name: | Water (10%) Supplier: Cayman Chemical Batch |
| No.: | 0634617-1 |

### Positive Controls

| | **without** metabolic activation | | **with** metabolic activation |
|---|---|---|---|
| Name | EMS, ethyl methanesulfonate | MMS, methyl methanesulfonate | B[a]P, benzo[a]pyrene |
| CAS no. | 62-50-0 | 66-27-3 | 50-32-8 |
| Supplier | Sigma-Aldrich | Sigma-Aldrich | Sigma-Aldrich |
| Batch no. | BCCF0900 | MKCL6261 | SLBW7628 |
| Dissolved in | cell culture medium | 0.9% NaCl | DMSO, dimethylsulfoxide; final concentration in RPMI medium: 1% |
| Final conc. | 200 and 300 µg/mL | 8 and 10 µg/mL | 3.5 µg/mL |

The dilutions of the positive control stock solutions were prepared on the day of experiment and used immediately. The stability of the positive control substances in solution is proven by the mutagenic response in the expected range.

### Test System

### The Cells

The L5178Y tk^{+/-} 3.7.2C cell line has been successfully used in in vitro experiments for many years. These cells are characterised by their high proliferation rate (10-12 h doubling time of the Eurofins Munich stock cultures) and their cloning efficiency, typically more than 50%. The cells obtain a near diploid karyotype (40 ± 2 chromosomes) and are heterozygous at the thymidine kinase (tk)locus.

To prevent high background counts arising from spontaneous mutation, tk^{-/-} cells can be eliminated by culturing in RPMI 1640 supplemented with: 9.0 µg/mL, hypoxanthine, 15.0 µg/mL thymidine,
22.5 µg/mL glycine, 0.1 µg/mL methotrexate.

The cells are resuspended in medium without methotrexate, but with thymidine, hypoxanthine, and glycine for 1-3 days.

Large stock cultures of the cleansed L5178Y tk^{+/-} 3.7.2C cell line are stored over liquid nitrogen (vapour phase) in the cell bank of Eurofins Munich. This allows the repeated use of the same cell batch in experiments. Each cell batch is routinely checked for an absence of mycoplasma.

Thawed stock cultures are maintained in plastic culture flasks in RPMI 1640 complete medium and sub-cultured on demand.

### Post-Mitochondrial Fraction (S9)

Substances may only develop mutagenic potential when they are metabolised by the mammalian organism. The cell line L5178Y tk^{+/-} 3.7.2C has an inadequate endogenous metabolic capacity and therefore an exogenous MA system is necessary . The most commonly used system is a co-factor- supplemented post-mitochondrial fraction (S9) prepared from livers of rodents.

The S9 fraction was prepared at Eurofins Munich. Male Wistar rats were induced with phenobarbital (80 mg/kg bw) and □-naphthoflavone (100 mg/kg bw) for three consecutive days by oral route. The preparation was performed according to Ames et al..

The following quality control determinations were performed:
a) Biological activity in the Salmonella typhimurium assay using 2-aminoanthracene and benzo[a]pyrene
b) Sterility test

A stock of the supernatant containing the S9 fraction was frozen in aliquots of 2 and 4 mL and stored at ≤-75 °C. The protein concentration in the S9 fraction was 35 mg/mL (Lot: 191121).

### Preparation of S9 Mix

The S9 mix preparation was performed according to Ames et al..

An appropriate quantity of S9 fraction was thawed and mixed with a co-factor solution to result in a final protein concentration of 0.75 mg/mL in the cultures. In 100 mM sodium phosphate buffer pH 7.4, the following concentrations were achieved: 8 mM MgCl2, 33 mM KCl, 5 mM Glucose-6-phosphate, 5 mM NADP. During the experiment the S9 mix was stored on ice.

### Experimental Design

### Exposure Concentrations

Criteria to determine the highest concentration were cytotoxicity, solubility in the test system, and changes in pH or osmolality. Cytotoxicity was determined with and without metabolic activation.

Several concentrations of the test item were set-up.

In STE⁽⁻⁾ STE⁽⁺⁾ LTE 5 mg/mL was selected as the highest concentration. Solvent vehicle or negative controls were tested in duplicate.

### Experimental Performance

For the STE 10⁷ cells were suspended in 11 mL RPMI medium with 5% horse serum (25 cm² flasks) and exposed to designated concentrations of the solved test item, in the presence or absence of MA. After 4 h, the solved test item was removed by centrifugation (200 xg, 7 min) and the cells were washed twice with PBS. Subsequently the cells were suspended in 30 mL complete culture medium and incubated for an expression and growth period of two days in total at 37 °C in 5% CO2/95% humidified air. The cell density was determined each day and, if necessary, adjusted to 3x10⁵ cells/mL.

For the LTE, 5x10⁶ cells were suspended in 25 mL RPMI medium with 7.5% horse serum (75 cm² flasks) and exposed to designated concentrations of the solved test item in the absence of MA. After 24 h, the solved test item was removed by centrifugation (200 xg, 7 min) and the cells were washed twice with PBS. Subsequently, 3x10⁵ cells/mL were suspended in 14 mL complete culture medium and incubated for an expression and growth period of 2 days at 37 °C in 5% CO2/95% humidified air. The cell density was determined each day and adjusted to 3x10⁵ cells/mL, if necessary.

After the expression period the relative cloning efficiency (RCE; percentage cloning efficiency of the test group in relation to the vehicle controls) of the cells was determined by seeding a statistical number of 1.6 cells/well in two 96-well plates. The cells were incubated for at least seven days at 37 °C in 5% CO2/95% humidified air. Analysis of the results was based on the number of cultures with cell growth (positive wells) and those without cell growth (negative wells) compared to the total number of cultures seeded. Relative suspension growth (RSG) and RTG (RTG = [RSG x RCE] / 100) of the treated cell cultures were calculated according to the method of Clive and Spector. Additionally, cultures were seeded in selective medium. Cells from each experimental group were seeded in four 96-well plates at a density of approximately 2000 cells/well in 200 µL selective medium with TFT. The plates were scored after an incubation period of 11 to 14 days at 37 °C in 5% CO2/95% humidified air.

The MF was calculated by dividing the number of TFT resistant colonies by the number of cells plated for selection, corrected for the plating efficiency of cells from the same culture grown in the absence of TFT. The Poisson distribution was used to calculate the plating efficiencies for cells cloned without and with TFT selection. Based on the null hypothesis of the Poisson distribution, the probable number of clones/well (P) is equal to -ln (negative wells/total wells) and the plating efficiency (PE) equals P/(number of cells plated per well). MF then was calculated as MF = (PE (cultures in selective medium)/PE (cultures in non-selective medium)). The MF is usually expressed as "mutants per 10⁶ viable cells" $Mutant frequency = \frac{- ln \left(\frac{negative wells}{total wells \left[selective medium\right]}\right)}{- ln \left(\frac{negative wells}{total wells \left[non-selective medium\right]}\right)} \times 800$

Suspension growth (SG) of the cell cultures reflects the number of times the cell number increases from the starting cell density. When conducting the STE, a two-day growth period was considered, when conducting the LTE, the treatment period of 24 h and the two-day growth period was considered. The RTG is the product of the RSG (calculated by comparing the SG of the test groups with the SG of the control) and the RCE for each culture: RTG = RSG x RCE / 100.

To specify the GEF the international workshop on genotoxicity testing (IWGT) analysed distributions of negative/vehicle mutant frequencies from ten laboratories. The GEF is defined as the mean of the negative/vehicle MF plus one standard deviation. Applying this definition to the collected data, the GEF is 126 mutants per 10⁶ cells for the microwell method.

The size of the colonies was characterised as follows: Small colonies approximately ≤ ¼ of well diameter. Large colonies approximately > ¼ of well diameter. Size is the key factor and morphology should be secondary.

### Cell Culture Media

Prior to the preparation of different media, horse serum (HS) was heat-inactivated for 30 min at 56 °C.

| | **complete culture medium** | **treatment medium** | **selective medium** |
|---|---|---|---|
| HS | 10% | STE: 5% | 20% |
| | | LTE: 7.5% | |
| penicillin/streptomycin | 100 U/100 µg/mL | 100 U/100 µg/mL | 100 U/100 µg/mL |
| sodium pyruvate | 1 mM | 1 mM | 1 mM |
| L-glutamine | 2 mM | 2 mM | 2 mM |
| HEPES | 25 mM | 25 mM | 25 mM |
| amphotericin B | 2.5 µg/mL | 2.5 µg/mL | 2.5 µg/mL |
| TFT | - | - | 5 µg/mL |

The selective agent (TFT) was obtained from Sigma-Aldrich (Lot No.: BCCD1996).

### Data recording

The generated data were recorded on the experimental design protocol. The results are presented in tabular form, including experimental groups with the test item, negative and positive controls. Individual colony counts for the treated and control groups are presented for both mutation induction and survival. Small colonies and large colonies were determined for the relevant test groups.

### Statistics

Only in cases where the GEF is exceeded:
The non-parametric Mann-Whitney test was applied to the mutation data to determine if there are statistical differences between the mutant frequencies of the treated test groups compared to the negative/solvent controls.

Only for full studies: A statistical trend analysis was performed.

### Acceptability of the Assay

A mutation assay is considered acceptable if it meets the criteria defined in current international guidelines and the current recommendations of the IWGT
- At least three out of four 96-well plates from the TFT selection experiment are analysable.
- The cloning efficiency of the negative or solvent controls is in the range 65%-120%.
- The spontaneous MF in the negative or solvent controls is in the range 50-170 mutants per 10⁶ cells.
- The cell number of the negative/solvent controls undergo 8-32-fold increase during a two-day growth period (STE) or 32-180-fold increase during a three-day growth period (LTE).
- The positive controls (MMS and B[a]P) for clastogenicity produce an induced MF (total MF minus concurrent negative control MF) of at least 300 mutants per 10⁶ cells with at least 40% of the colonies being small colonies or with an induced small colony MF of at least 150 mutants per 10⁶ cells.
- The RTG must be greater than or equal to 10%.

### Evaluation of Results

The test item is considered mutagenic if the following criteria are met:
- The induced MF meets or exceeds the GEF
- The RTG must be greater than or equal to 10%.
- A concentration-dependent increase in MF is detected using an appropriate statistical trend analysis (not for limit study).

Besides, combined with a positive effect in the MF, an increased occurrence of small colonies (≥ 40% of total colonies) is an indicator of potentially clastogenic effects.

A test item is considered negative if the induced MF is below the GEF or the trend of the test is negative.

### Interpretation of Results

There is no requirement for verification of a clear positive response.

Equivocal results should be clarified by further testing preferably using a modification of experimental conditions.

Negative results need to be confirmed on a case-by-case basis.

Study parameters which might be changed are concentration spacing, duration of treatment (LTE without MA) or metabolic activation conditions.

### Results of STE⁽⁻⁾

**Table 4: STE⁽⁻⁾ - Toxicity Data**

| **Test Group** | **Conc. [mg/mL]** | **No. of cells 4 h after treatment** | **No. of cells 24 h after treatment** | **No. of cells 48 h after treatment** | **SG^{a}** | **RSG^{b} [%]** | **RCE^{c} [%]** | **RTG^{d} [%]** |
|---|---|---|---|---|---|---|---|---|
| NC | 0 | 422 000 | 1 360 000 | 1 360 000 | 18 | 94 | 89 | 84 |
| | 0 | 398 000 | 1 480 000 | 1 260 000 | 19 | 95 | 109 | 103 |
| SC | 0 | 426 000 | 1 350 000 | 1 380 000 | 19 | 100 | 100 | 100 |
| | 0 | 380 000 | 1 480 000 | 1 400 000 | 21 | | | |
| Test Item | 0.25 | 401 000 | 1 490 000 | 1 060 000 | 16 | 80 | 112 | 90 |
| | 0.5 | 279 000 | 1 470 000 | 1 360 000 | 20 | 102 | 114 | 116 |
| | 1 | 378 000 | 1 410 000 | 1 470 000 | 21 | 105 | 81 | 85 |
| | 3 | 316 000 | 1 300 000 | 1 400 000 | 18 | 93 | 90 | 84 |
| | 5 | 404 000 | 1 460 000 | 1 310 000 | 19 | 97 | 81 | 78 |
| EMS | 300 µg/mL | 403 000 | 1 210 000 | 1 330 000 | 16 | 82 | 60 | 49 |
| MMS | 10 pg/mL | 392 000 | 1 070 000 | 1 380 000 | 15 | 75 | 85 | 64 |

| | |
|---|---|
| NC: | Negative control |
| SC: | Solvent control (Water (10%)) |
| a: | Suspension growth. SG = [((value 24 h x 30) / 1 x 10⁷) x ((value 48 h x 20) / (value 24 h* x 20))]; *: for value 24 h > 3 x 10⁵ then value 24 h = 3 x 10⁶ |
| b: | Relative suspension growth, RSG = [(value SG / value SG of corresponding controls) x 100) |
| c: | Relative cloning efficiency. RCE = [(CE _{test group} / CE _{of corresponding controls}) x 100]; Cloning efficiency, CE = ((-ln (((96 - (mean Plate 1, Plate 2)) / 96)) / 1.6) x 100) |
| d: | Relative total growth, RTG = (RSG x RCE) / 100 |
| EMS: | Ethyl methanesulfonate |
| MMS: | Methyl methanesulfonate |

**Table 5: STE⁽⁻⁾ - Mutagenicity Data**

| **Test Group** | **Conc. [mg/mL]** | **Cloning Efficiency (CE)** | | | **Mutagenicity Data** | | | | | | |
|---|---|---|---|---|---|---|---|---|---|---|---|
| | | **Plate 1°** | **Plate 2°** | **CE [%]^{f}** | **Number of cultures/96 wells** | | | | | **MF^{g} (mutants/10⁶ cells]** | **IMF^{h} [mutants/10⁶ cells]** |
| | | | | | **Plate 1°** | **Plate 2** | **Plate 3°** | **Plate 4°** | **Mean** | | |
| NC | 0 | 59 | 65 | 65 | 24 | 16 | 19 | 17 | 19.0 | 171 | / |
| | 0 | 72 | 66 | 79 | 16 | 14 | 22 | 20 | 18.0 | 131 | / |
| SC | 0 | 69 | 66 | 76 | 16 | 23 | 21 | 12 | 18.0 | 138 | / |
| | 0 | 67 | 62 | 70 | 27 | 10 | 19 | 21 | 19.3 | 163 | / |
| Test Item | 0.25 | 68 | 72 | 82 | 22 | 22 | 12 | 18 | 18.5 | 132 | -18,4 |
| | 0.5 | 81 | 60 | 83 | 25 | 24 | 19 | 21 | 22.3 | 159 | 9,1 |
| | 1 | 60 | 57 | 59 | 19 | 19 | 17 | 16 | 17.8 | 174 | 23,8 |
| | 3 | 68 | 57 | 66 | 17 | 18 | 19 | 24 | 19.5 | 173 | 22,7 |
| | 5 | 55 | 62 | 59 | 19 | 22 | 18 | 18 | 19.3 | 191 | 40,3 |
| EMS | 300 µg/mL | 44 | 53 | 44 | 68 | 69 | 74 | 72 | 70.8 | 1 524 | 1 373,3 |
| MMS | 10 µg/mL | 64 | 57 | 62 | 64 | 51 | 66 | 62 | 60.8 | 816 | 665,4 |

| | |
|---|---|
| NC: | Negative control |
| SC: | Solvent control (Water (10%)) |
| e: | Number of cultures with cell growth. |
| f: | Cloning efficiency, CE = ((-ln (((96 - (mean Plate 1, Plate 2)) / 96)) / 1.6) x 100) |
| g: | Mutant frequency; MF = {-ln [negative cultures/total wells (selective medium)] / -ln [negative cultures/total wells (non-selective medium)]}×800 |
| h: | Induced mutant frequency, IMF = mutant frequency sample - mean value mutant frequency corresponding controls |
| EMS: | Ethyl methanesulfonate |
| MMS: | Methyl methanesulfonate |

**Table 6: STE⁽⁻⁾ - Colony Sizing**

| **Test Group*** | **Cone. [mg/mL]** | **Wells with at least 1 colony** | **Large colonies** | **Small colonies** | **% small colonies** |
|---|---|---|---|---|---|
| NC | 0 | 76 | 62 | 14 | 18 |
| | 0 | 72 | 59 | 13 | 18 |
| SC | 0 | 72 | 56 | 16 | 22 |
| | 0 | 77 | 63 | 14 | 18 |
| EMS | 300 µg/mL | 283 | 245 | 38 | 13 |
| MMS | 10 µg/mL | 243 | 122 | 121 | 50 |

| | |
|---|---|
| NC**:** | Negative control |
| SC: | Solvent control (Water (10%)) |
| EMS: | Ethyl methanesulfonate |
| MMS: | Methyl methanesulfonate |
| *: | Based on the non-mutagenic effects of the test item, an assessment of clastogenicity was not feasible. |

### Results of STE⁽⁺⁾

**Table 7: STE⁽⁺⁾ - Toxicity Data**

| **Test Group** | **Cone. [mg/mL]** | **No. of cells 4 h after treatment** | **No. of cells 24 h after treatment** | **No. of cells 48 h after treatment** | **SG^{a}** | **RSG^{b} [%]** | **RCE^{c} [%]** | **RTG^{d}[%]** |
|---|---|---|---|---|---|---|---|---|
| NC | 0 | 423 000 | 1 240 000 | 1 420 000 | 18 | 104 | 102 | 106 |
| | 0 | 464 000 | 1 340 000 | 1 340 000 | 18 | 106 | 99 | 105 |
| SC | 0 | 396 000 | 1 320 000 | 1 230 000 | 16 | 100 | 100 | 100 |
| | 0 | 436 000 | 1 320 000 | 1 330 000 | 18 | | | |
| Test Item | 0.25 | 421 000 | 1 280 000 | 1 280 000 | 16 | 97 | 95 | 92 |
| | 0.5 | 384 000 | 1 260 000 | 1 230 000 | 15 | 92 | 113 | 104 |
| | 1 | 410 000 | 1 410 000 | 1 390 000 | 20 | 116 | 110 | 127 |
| | 3 | 406 000 | 1 380 000 | 1 230 000 | 17 | 100 | 115 | 115 |
| | 5 | 423 000 | 1 390 000 | 1 310 000 | 18 | 108 | 64 | 69 |
| B[a]P | 3.5 µg/mL | 386 000 | 743 000 | 1 240 000 | 9 | 55 | 71 | 39 |

| | |
|---|---|
| NC: | Negative control |
| SC: | Solvent control (Water (10%)) |
| a: | Suspension growth, SG = [((value 24 h x 30) / 1 x 10⁷) x ((value 48 h x 20) / (value 24 h* x 20))]; *: for value 24 h > 3 x 10⁶ then value 24 h = 3 x 10⁵ |
| b: | Relative suspension growth, RSG = [(value SG / value SG of corresponding controls) x 100] |
| c: | Relative cloning efficiency, RCE = [(CE _{test group} / CE _{of corresponding controls}) x 100]; Cloning efficiency, CE = ((-ln (((96 - (mean Plate 1, Plate 2)) / 96)) / 1.6) x 100) |
| d: | Relative total growth, RTG = (RSG x RCE) / 100 |
| B[a]P: | Benzo[a]pyrene |

**Table 8: STE⁽⁺⁾ - Mutagenicity Data**

| **Test Group** | **Conc. [mg/mL]** | **Cloning Efficiency (CE)** | | | **Mutagenicity Data** | | | | | | |
|---|---|---|---|---|---|---|---|---|---|---|---|
| | | **Plate 1°** | **Plate 2°** | **CE [%]^{f}** | **Number of cultures/96 wells** | | | | | **MF^{g} [mutants/10⁶ cells]** | **IMF^{h} [mutants/10⁶ cells]** |
| | | | | | **Plate 1°** | **Plate 2°** | **Plate 3°** | **Plate 4°** | **Mean** | | |
| NC | 0 | 72 | 66 | 79 | 22 | 20 | 19 | 18 | 19.8 | 145 | / |
| | 0 | 68 | 68 | 77 | 12 | 20 | 12 | 16 | 15.0 | 111 | / |
| SC | 0 | 70 | 71 | 83 | 20 | 16 | 14 | 16 | 16.5 | 114 | / |
| | 0 | 69 | 63 | 73 | 14 | 15 | 19 | 19 | 16.8 | 132 | / |
| Test Item | 0.25 | 66 | 67 | 74 | 23 | 20 | 17 | 24 | 21.0 | 168 | 45 |
| | 0.5 | 68 | 77 | 88 | 23 | 17 | 14 | 20 | 18.5 | 122 | -1 |
| | 1 | 71 | 72 | 85 | 22 | 20 | 27 | 20 | 22.3 | 155 | 32 |
| | 3 | 73 | 73 | 89 | 17 | 19 | 11 | 22 | 17.3 | 112 | -11 |
| | 5 | 52 | 53 | 49 | 21 | 17 | 19 | 22 | 19.8 | 233 | 110 |
| B[a]P | 3.5 µg/mL | 56 | 57 | 56 | 52 | 60 | 56 | 56 | 56.0 | 791 | 668 |

| | |
|---|---|
| NC: | Negative control |
| SC: | Solvent control (Water (10%)) |
| e: | Number of cultures with cell growth. |
| f: | Cloning efficiency, CE = ((-ln (((96 - (mean Plate 1, Plate 2)) / 96)) / 1.6) x 100) |
| g: | Mutant frequency; MF = {-ln [negative cultures/total wells (selective medium)]/ -ln [negative cultures/total wells (non-selective medium)]}x800 |
| h: | induced mutant frequency, IMF = mutant frequency sample - mean value mutant frequency corresponding controls |
| B[a]P: | Benzo[a]pyrene |

### STE⁽⁺⁾ - Colony Sizing

| **Test Group*** | **Conc. [mg/mL]** | **Wells with at least 1 colony** | **Large colonies** | **Small colonies** | **% small colonies** |
|---|---|---|---|---|---|
| NC | 0 | 79 | 66 | 13 | 16 |
| | 0 | 60 | 48 | 12 | 20 |
| SC | 0 | 66 | 57 | 9 | 14 |
| | 0 | 67 | 59 | 8 | 12 |
| B[a]P | 3.5 µg/mL | 224 | 132 | 92 | 41 |

| | |
|---|---|
| NC: | Negative control |
| SC: | Solvent control (Water (10%)) |
| B[a]P: | Benzo[a]pyrene |
| *: | Based on the non-mutagenic effects of the test item, an assessment of clastogenicity was not feasible. |

### Results of LTE Table 10: LTE - Toxicity Data

| **Test Group** | **Conc. [mg/mL]** | **No. of cells 24 h after treatment** | **No. of cells 48 h after treatment** | **No. of cells 72 h after treatment** | **SG^{a}** | **RSG^{b} [%]** | **RCE^{c} [%]** | **RTG^{d} [%]** |
|---|---|---|---|---|---|---|---|---|
| NC | 0 | 1 730 000 | 1 260 000 | 1 330 000 | 161 | 94 | 84 | 79 |
| | 0 | 1 740 000 | 1 290 000 | 1 540 000 | 192 | 112 | 101 | 113 |
| SC | 0 | 1 620 000 | 1 430 000 | 1 290 000 | 166 | 100 | 100 | 100 |
| | 0 | 1 710 000 | 1 310 000 | 1 430 000 | 178 | | | |
| Test Item | 0.25 | 1 660 000 | 1 350 000 | 1 390 000 | 113 | 101 | 87 | 88 |
| | 0.5 | 1 600 000 | 1 380 000 | 1 310 000 | 161 | 93 | 112 | 105 |
| | 1 | 1 420 000 | 1 420 000 | 1 410 000 | 158 | 92 | 101 | 93 |
| | 3 | 973 000 | 828 000 | 1 320 000 | 59 | 34 | 103 | 35 |
| | 5 | 777 000 | 907 000 | 1 330 000 | 52 | 30 | 86 | 26 |
| EMS | 200 µg/mL | 1 150 000 | 1 170 000 | 1 160 000 | 87 | 50 | 56 | 28 |
| MMS | 10 µg/mL | 1 260 000 | 1 360 000 | 1 440 000 | 137 | 80 | 60 | 48 |

| | |
|---|---|
| NC: | Negative control |
| SC: | Solvent control (Water (10%)) |
| a: | Suspension Growth, SG = [((value 24hx25) / 5x10⁶) x ((value 48hx14)/(value 24h*x14)) x ((value 72hx14)/( value 48h*x14))]; *: for value 24 h and 48 h > 3 x 10⁵ then value 24 h and 48 h = 3 x 10⁵ |
| b: | Relative suspension growth, RSG = ((value SG / value SG of corresponding controls) x 100) |
| c: | Relative Cloning Efficiency, RCE = [(CE _{test group} / CE _{of corresponding controls}) x 100]; Cloning efficiency, CE = ((-ln (((96 - (mean Plate 1, Plate 2)) / 96)) / 1.6) x 100) |
| d: | Relative total growth, RTG = (RSG x RCE) / 100 |
| EMS: | Ethyl methanesulfonate |
| MMS: | Methyl methanesulfonate |

**Table 11: LTE - Mutagenicity Data**

| **Test Group** | **Conc. [mg/mL]** | **Cloning Efficiency (CE)** | | | **Mutagenicity Data** | | | | | | |
|---|---|---|---|---|---|---|---|---|---|---|---|
| | | **Plate 1°** | **Plate 2°** | **CE [%]^{f}** | **Number of cultures/96 wells** | | | | | **MF^{g} [mutants/10** ⁶ **cells)** | **IMF^{h} [mutants/10 ⁶ cells]** |
| | | | | | **Plat e 1°** | **Plat e 2°** | **Plat e 3°** | **Plat e 4°** | **Mea n** | | |
| NC | 0 | 73 | 75 | 92 | 10 | 13 | 14 | 12 | 12.3 | 74 | / |
| | 0 | 79 | 80 | 110 | 16 | 16 | 8 | 13 | 13.3 | 68 | / |
| SC | 0 | 76 | 76 | 98 | 10 | 11 | 13 | 14 | 12.0 | 68 | / |
| | 0 | 88 | 76 | 120 | 16 | 7 | 6 | 12 | 10.3 | 47 | / |
| Test Item | 0.25 | 73 | 77 | 95 | 14 | 11 | 17 | 21 | 15.8 | 95 | 37.1 |
| | 0.5 | 83 | 82 | 123 | 11 | 15 | 12 | 5 | 10.8 | 49 | -9.0 |
| | 1 | 78 | 81 | 110 | 10 | 15 | 15 | 15 | 13.8 | 70 | 12.6 |
| | 3 | 76 | 84 | 112 | 14 | 12 | 19 | 11 | 14.0 | 71 | 12.9 |
| | 5 | 70 | 79 | 94 | 11 | 21 | 16 | 24 | 18.0 | 112 | 54.3 |
| EMS | 200 µg/mL | 59 | 61 | 61 | 91 | 90 | 91 | 92 | 91.0 | 2.418 | 2.360.7 |
| MMS | 10 µg/mL | 66 | 59 | 66 | 59 | 55 | 52 | 56 | 55.5 | 657 | 599.5 |

| | |
|---|---|
| NC: | Negative control |
| SC: | Solvent control (Water (10%)) |
| e: | Number of cultures with cell growth |
| f: | Cloning Efficiency, CE = ((-ln (((96 - (mean Plate 1. Plate 2)) / 96)) / 1.6) x 100) |
| g: | Mutant frequency, MF = {-In [negative cultures/total wells (selective medium)) /-ln [negative cultures/total wells (non-selective medium)]}×800 |
| h: | Induced mutant frequency. IMF = mutant frequency sample - mean value mutant frequency corresponding controls |
| EMS: | Ethyl methanesulfonate |
| MMS: | Methyl methanesulfonate |

**Table 12: LTE - Colony Sizing**

| **Test Group*** | **Conc. [mg/mL]** | **Wells with at least 1 colony** | **Large colonies** | **Small colonies** | **% small colonies** |
|---|---|---|---|---|---|
| NC | 0 | 49 | 39 | 10 | 20 |
| | 0 | 53 | 41 | 12 | 23 |
| SC | 0 | 48 | 37 | 11 | 23 |
| | 0 | 41 | 33 | 8 | 20 |
| EMS | 200 µg/mL | 364 | 343 | 21 | 6 |
| MMS | 10 µg/mL | 222 | 116 | 106 | 48 |

| | |
|---|---|
| NC: | Negative control |
| SC: | Solvent control (Water (10%)) |
| EMS: | Ethyl methanesulfonate |
| MMS: | Methyl methanesulfonate |
| *: | Based on the non-mutagenic effects of the test item, an assessment of clastogenicity was not feasible. |

### Discussion

The test item BIO ACTIVE COLL was assessed for its potential to induce mutations at the mouse lymphoma thymidine kinase locus (tk^{+/-}) using the L5178Y cell line.

For the short-term experiments (STE), the solved test item was incubated with cells for 4 hours. The STEs were performed independently: with (STE⁽⁺⁾) and without (STE⁽⁻⁾) metabolic activation. For the long-term experiment (LTE), the solved test item was incubated with cells for 24 hours. The LTE was performed without metabolic activation.

The solved test item was investigated at the following concentrations:
STE⁽⁻⁾: 0.25, 0.50, 1, 3 and 5 mg/mL
STE⁽⁺⁾: 0.25, 0.50, 1, 3 and 5 mg/mL
LTE: 0.25, 0.50, 1, 3 and 5 mg/mL

### Test Item Properties

The measured pH value of the solved test item was within the physiological range. No precipitation of the solved test item was noted in the experiments.

### Toxicity

In STE⁽⁻⁾ STE⁽⁺⁾ LTE 5 mg/mL was selected as the highest concentration. No growth inhibition was observed in STE⁽⁻⁾ (Table 4).

Growth inhibition was observed in STE⁽⁺⁾ and LTE. In STE⁽⁺⁾, the relative total growth (RTG) was 69% for highest concentration evaluated (Table 7). In LTE, the relative total growth (RTG) was 26% for highest concentration evaluated (Table 10).

### Mutagenicity

The induced mutant frequencies obtained from all experiments were compared to the GEF. The global evaluation factor (GEF) was not exceeded by the induced mutant frequency. The GEF is defined as the mean of the negative/vehicle mutant frequency plus one standard deviation; data are gathered from ten laboratories. For the microwell method, the GEF was defined to be 126 mutants/10⁶ cells. Criterion for mutagenicity is the extension of the GEF by the induced mutant frequency as well as a concentration-dependent increase in mutant frequency. The positive controls EMS (200 and 300 µg/mL), MMS (8 and 10 µg/mL) and B[a]P (3.5 µg/mL) showed distinct responses in mutation frequency, thus demonstrating the ability of the test system to detect potentially mutagenic effects.

All criteria of validity were met (10.9), except of the mutant frequencies for the 1 negative control of STE⁽⁻⁾: 171 and 1 solvent control of LTE:47 (acceptance range of 50-170 mutants/10⁶ cells, according to the IWGT criteria). Nevertheless, the negative control and solvent control values were considered acceptable for inclusion in the historical control data set (Table 13), as they were only slightly outside of the historical range and no technical reason or human failure was determined.

No biologically relevant increase of mutants was found in any experiments, the GEF was not exceeded (Table 5, Table 8, Table 11)

### Clastogenicity

The increased occurrence of small colonies (defined by slow growth and morphological alteration of the cell clone) in combination with a GEF exceeding induced mutant frequency is an indication of potentially clastogenic effects. Colony size was counted for all concentrations of the test item and for the negative and positive controls.

The positive controls MMS and B[a]P induced a significant increase in mutant frequency and a biologically significant increase of small colonies (≥ 40%), thus confirming the ability of the test system to indicate potentially clastogenic effects (Table 6, Table 9, Table 12). In all experiments the percentage of small colonies in the negative and solvent controls was lower than 40%.

Based on the non-mutagenic effects of BIO ACTIVE COLL, an assessment of clastogenicity was not feasible.

### Conclusion

In conclusion, in this in vitro Mammalian Cell Gene Mutation Assay (Thymidine Kinase Locus/tk+/-) in L5178Y Mouse Lymphoma Cells, under the experimental conditions reported, the test item BIO ACTIVE COLL is considered to be non-mutagenic.

### EXPERIMENTAL TEST 6

### REVERSE MUTATION ASSAY USING BACTERIA (SALMONELLA TYPHIMURIUM AND ESCHERICHIA COLI) WITH BIO ACTIVE COLL

### Summary Results

In order to investigate the potential of BIO ACTIVE COLL for its ability to induce gene mutations the plate incorporation test was performed with the Salmonella typhimurium strains TA98, TA100, TA1535, TA1537 and E. coli WP2 uvrA (pKM 101).

In the experiment several concentrations of the test item were used. The assay was conducted with and without metabolic activation. The concentrations, including the controls, were tested in triplicate. The following concentrations of the test item were prepared:
31.6, 100, 316, 1000, 2500 and 5000 µg/plate

No precipitation of the test item was observed in any tester strain used (with and without metabolic activation).

No toxic effects of the test item were noted in any of the five tester strains used up to the highest dose group evaluated with and without metabolic activation.

No biologically relevant increases in revertant colony numbers of any of the five tester strains were observed following treatment with BIO ACTIVE COLL at any concentration level, neither in the presence nor absence of metabolic activation. All criteria of validity were met (see).

### Conclusion

In conclusion, it can be stated that during the described mutagenicity test and under the experimental conditions reported, BIO ACTIVE COLL did not cause gene mutations by base pair changes or frameshifts in the genome of the tester strains used.

Therefore, BIO ACTIVE COLL is considered to be non-mutagenic in this bacterial reverse mutation assay.

### Aim of the Study

Bacterial reverse mutation assays use amino acid requiring strains of Salmonella typhimurium and Escherichia coli (E. coli) to detect point mutations, which involve substitution, addition or deletion of one or a few DNA base pairs. The principle of these bacterial reversion assays is that they detect mutations which functionally reverse mutations present in the tester strains and restore the capability to synthesize an essential amino acid.

The purpose of this study is to establish the potential of the test item to induce gene mutations in bacteria by means of a S. typhimurium and E. coli reverse mutation assay. Further confirmatory testing in the case of clearly negative or positive test results is not usually needed. Equivocal results should be clarified by further testing preferably using a modification of experimental conditions. Modification of study parameters to extend the range of conditions assessed should be considered in follow-up experiments. Study parameters that might be modified include the concentrations spacing and / or the method of treatment (pre-incubation method). In case of severe toxicity of the test item or the use of
e.g. ethanol, acetone or tetrahydrofuran as the most appropriate solvent, the confirmatory experiment is carried out according to the plate incorporation method with a different spacing between dose levels.

Due to clear negative results in the first experiment of this study no independent repetition was performed, as described in 10993-33.

The S. typhimurium histidine (his) reversion system and the E. coli tryptophan (trp) reversion systems measure his⁻ ->his⁺ reversions and trp→trp⁺. The S. typhimurium strains are constructed to differentiate between base pair (TA100, TA1535) and frameshift (TA98, TA1537) mutations. The E. coli strain detects only base substitution mutagens.

These assays directly measure heritable DNA mutations of a type which is associated with adverse effects. Point mutations are the cause of many human genetic diseases and there is substantial evidence that somatic cell point mutations in oncogenes and tumour suppressor genes are involved in cancer in humans and experimental systems. The tester strains have several features that make them more sensitive for the detection of mutations. The specificity of the strains can provide useful information on the types of mutations that are induced by mutagenic agents.

According to the direct plate incorporation method the bacteria are exposed to the test item with and without metabolic activation and plated on selective medium. After a suitable period of incubation, revertant colonies are counted.

At least five different concentrations of the test item are tested with approximately half log (i.e. √10) intervals between test points for an initial test. Narrower spacing between dose levels may be appropriate when a dose response is investigated. For soluble, nontoxic test compounds the recommended maximum test concentration is 5 mg/plate or 5 µL/plate.

To validate the test, reference mutagens are tested in parallel to the test item.

### Justification for the Selection of the Test System

The OECD Guideline for Testing of Chemicals, Section 4, No. 471 - Bacterial Reverse Mutation Test - recommends using a combination of S. typhimurium strains TA98, TA100, TA1535, TA1537 and TA102 or E. coli WP2 uvrA (pKM101).

### MATERIALS AND METHODS

### Characterisation of the Test Item

The identity of the test item was inspected upon delivery at the test facility (e.g. test item name, batch no. and additional data were compared with the label) based on the following specifications provided by the sponsor. The following listed information applies to the sample as received.

| | |
|---|---|
| Name: | BIO ACTIVE COLL |
| Lot No.: | 03/21 |
| Code: | TYPE 1 PROFIL |
| Manufacturing Date: | November 2021 |
| Expiry Date: | not applicable |
| Storage Conditions: | at room temperature, cool dry place, protected from light |
| Type of Material: | Natural Polymer |
| Sterility: | sterile |
| Sterilization Procedure: | γ-irradiation |
| Safety Precautions: | The routine hygienic procedures were sufficient to assure personnel health and safety. |

### Preparation of the Test Item

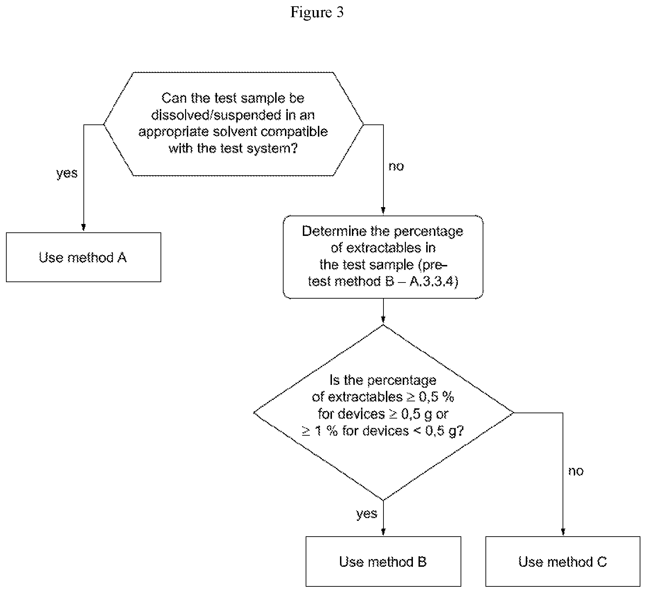

The test item was prepared in compliance to ISO 10993-3: 2014 "Tests for genotoxicity, carcinogenicity and reproductive toxicity" . The sample preparation should follow the decision tree in the Figure 3. This figure diagrams the decision process used to select the extraction method (method A, B or C).

If the test sample can be dissolved or suspended in an appropriate solvent within the test system, the test sample can be applied directly to the test system (Method A) at a maximum concentration of 5 mg/mL (in vitro mammalian test system) or 5 mg/plate (bacterial reverse mutation assay).

Since the test item can be dissolved (see Eurofins Munich Study 150459 "In vitro Cytotoxicity Assay: Evaluation of Materials for Medical Devices by Extraction Method and XTT Dye with BIO ACTIVE COLL") Method A was chosen.

The test item was dissolved in A. dest., processed by ultrasound for 5 min at 37 °C and diluted prior to treatment. The solvent was compatible with the survival of the bacteria and the S9 activity.

### Controls

Negative as well as positive controls were included in the experiment. Strain specific positive controls were included in the assay, which demonstrated the effective performance of the test.

### Negative/Solvent Controls

Negative controls (A. dest., Eurofins Munich, Lot No. 220218, 220304) were treated in the same way as all dose groups.

### Positive Controls

### Without metabolic activation

| | |
|---|---|
| Tester Strains: | S. typhimurium: TA100, TA1535 |
| Name: | NaN3; sodium azide |
| CAS No.: | 26628-22-8 |
| Supplier: | Sigma |
| Batch No.: | STBF8665V |
| Dissolved in: | A. dest. |
| Concentration: | 10 µg/plate |
| Tester Strains: | S. typhimurium: TA98, TA1537 |
| Name: | 4-NOPD; 4-nitro-o-phenylene-diamine |
| CAS No.: | 99-56-9 |
| Supplier: | Sigma |
| Batch No.: | MKCF1418 |
| Dissolved in: | DMSO |
| Concentrations: | 10 µg/plate for TA98, 40 µg/plate for TA1537 |
| Tester Strain: | E. coli WP2 uvrA (pKM101) |
| Name: | MMS; methylmethanesulfonate |
| CAS No.: | 66-27-3 |
| Supplier: | Sigma |
| Batch No.: | MKCG1346 |
| Dissolved in: | A. dest. |
| Concentration: | 1 µL/plate |

### With metabolic activation

| | |
|---|---|
| Tester Strains: | S. typhimurium: TA98, TA100, TA1535, TA1537 and E. coli WP2 uvrA (pKM101) |
| Name: | 2-AA; 2-aminoanthracene |
| CAS No.: | 613-13-8 |
| Supplier: | Alfa Aesar |
| Batch No.: | 102181359-A |
| Dissolved in: | DMSO |
| Concentrations: | 2.5 µg/plate for TA98, TA100, TA1535 and TA1537; 10 µg/plate for E. coli WP2 uvrA (pKM101) |

The stability of the positive control substances in solution is unknown but a mutagenic response in the expected range is sufficient evidence of biological stability.

### Test System

### Bacteria

Four strains of S. typhimurium and one strain of E. coli with the following characteristics are used: TA98:
Four strains of *S*. *typhimurium* and one strain of *E. coli* with the following characteristics are used:

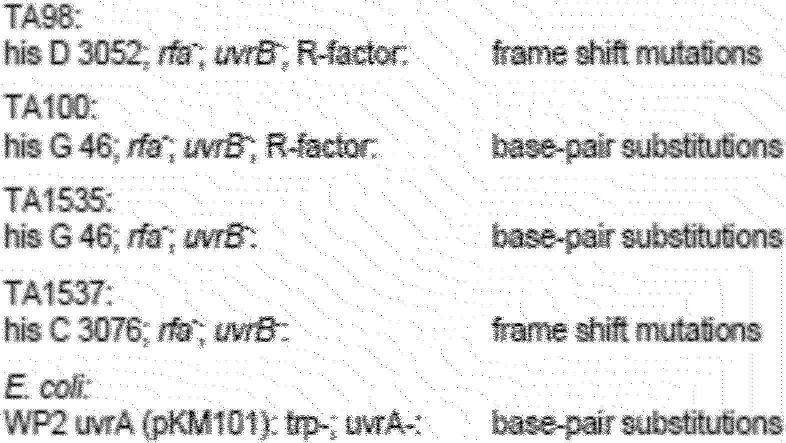

Tester strains TA98, TA1535 and E. coli were obtained from MOLTOX, INC., NC 28607, USA. Tester strains TA100 and TA1537 were obtained from Xenometrix AG, Switzerland. They were stored as stock cultures in ampoules with nutrient broth (OXOID) supplemented with DMSO (approx. 8% v/v) over liquid nitrogen.

All Salmonella strains contain mutations in the histidine operon, thereby imposing a requirement for histidine in the growth medium. They contain the deep rough (rfa) mutation, which deletes the polysaccharide side chain of the lipopolysaccharides of the bacterial cell surface. This increases cell permeability of larger substances. The other mutation is a deletion of the uvrB gene coding for a protein of the DNA nucleotide excision repair system resulting in an increased sensitivity in detecting many mutagens. This deletion also includes the nitrate reductase (chl) and biotin (bio) genes (bacteria require biotin for growth).

The tester strains TA98, TA100 and E. coli contain the R-factor plasmid, pkM101. These strains are reverted by a number of mutagens that are detected weakly or not at all with the non R-factor parent strains. pkM101 increases chemical and spontaneous mutagenesis by enhancing an error-prone DNA repair system which is normally present in these organisms.

The tester strain E. coli WP2 uvrA (pKM101) carries the defect in one of the genes for tryptophan biosynthesis. Tryptophan-independent mutants (revertants) can arise either by a base change at the site of the original alteration or by a base change elsewhere in the chromosome so that the original defect is suppressed. This second possibility can occur in several different ways so that the system seems capable of detecting all types of mutagens which substitute one base for another. Additionally, the strain is deficient in the DNA nucleotide excision repair system.

The properties of the S. typhimurium and E. coli strains with regard to membrane permeability, ampicillin- and tetracycline-resistance as well as normal spontaneous mutation rates are checked regularly according to Ames et al. In this way it is ensured that the experimental conditions set up by Ames are fulfilled.

### Preparation of Bacteria

Samples of each tester strain were grown by culturing for 12 h at 37 °C in S. typhimurium medium (Nutrient Broth) and E. coli medium (Luria Bertani), respectively, to the late exponential or early stationary phase of growth (approx. 10⁹ cells/mL).

The S. typhimurium medium (Nutrient Broth) contains per litre of purified water:

| | |
|---|---|
| 8 g | Nutrient Broth |
| 5 g | NaCl |

The E. coli medium (Luria Bertani) contains per litre of purified water:

| | |
|---|---|
| 10 g | tryptone |
| 10 g | NaCl |
| 5 g | yeast extract |

A solution of 125 µL ampicillin (10 mg/mL) (TA98, TA100, E. coli WP2 uvrA (pKM101)) was added in order to retain the phenotypic characteristics of the strain.

### Agar Plates

The Vogel-Bonner Medium E agar plates with 2% glucose used in the Ames test were prepared by Eurofins Munich or provided by an appropriate supplier. Quality controls were performed.

Vogel-Bonner-salts contain per litre of purified water:

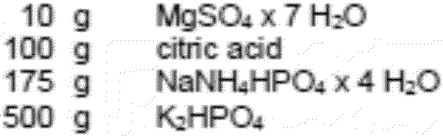

Sterilisation was performed for 20 min at 121 °C in an autoclave. Vogel-Bonner Medium E agar plates contain per litre of purified water:

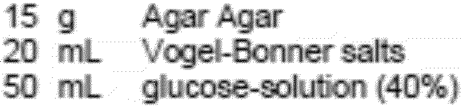

Sterilisation was performed for 20 min at 121 °C in an autoclave.

### Overlay Agar

The overlay agar contains per litre of purified water:
*S*. *typhimurium:* 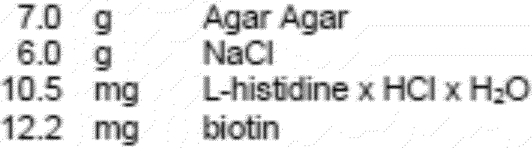
*E. coli:*

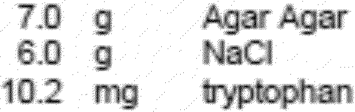

Sterilisation was performed for 20 min at 121 °C in an autoclave.

### Mammalian Microsomal Fraction S9 Mix

The bacteria most commonly used in these reverse mutation assays do not possess the enzyme system which, in mammals, is known to convert promutagens into active DNA damaging metabolites. In order to overcome this major drawback an exogenous metabolic system was added in the form of mammalian microsome enzyme activation mixture.

### S9 Homogenate

The S9 liver microsomal fraction was prepared at Eurofins Munich. Male Wistar rats were induced with phenobarbital (80 mg/kg bw) and β-naphthoflavone (100 mg/kg bw) for three consecutive days by oral route.

The following quality control determinations are performed:
a) Biological activity in the Salmonella typhimurium assay using 2-aminoanthracene and benzo[a]pyrene
b) Sterility Test

A stock of the supernatant containing the microsomes was frozen in aliquots of 2 and 4 mL and stored at ≤-75 °C.

The protein concentration in the S9 preparation (Lot: 191121) was 35.0 mg/mL.

### Preparation of S9 Mix

The S9 mix preparation was performed according to Ames et al.

100 mM of sodium-ortho-phosphate-buffer, pH 7.4, was ice-cold added to the following pre-weighed sterilised reagents to give final concentrations in the S9 mix of:

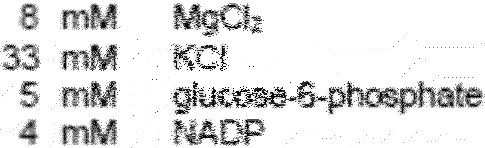

This solution was mixed with the liver 9000 x g supernatant fluid in the following proportion:

| | |
|---|---|
| co-factor solution | 9.5 parts |
| liver preparation | 0.5 parts |

During the experiment the S9 mix was stored on ice.

### S9 Mix Substitution Buffer

The S9 mix substitution buffer was used in the study as a replacement for S9 mix, without metabolic activation (-S9).

Phosphate-buffer (0.2 M) contains per litre of purified water:

| | |
|---|---|
| 0.2 M NaH2PO4 x H₂O | 120 mL |
| 0.2 M Na2HPO4 | 880 mL |

The two solutions were mixed and the pH was adjusted to 7.4. Sterilisation was performed for 20 min at 121 °C in an autoclave.

This 0.2 M phosphate-buffer was mixed with 0.15 M KCl solution (sterile) in the following proportion:

| | |
|---|---|
| 0.2 M phosphate-buffer | 9.5 parts |
| 0.15 M KCl solution | 0.5 parts |

This S9 mix substitution buffer was stored at 4 °C.

### Experimental Design

### Pre-Experiment for Toxicity

The toxicity of the test item was determined with tester strains TA98 and TA100 in a pre-experiment. Eight concentrations were tested for toxicity and induction of mutations with three plates each. The experimental conditions in this pre-experiment were the same as described below for the main experiment (plate incorporation test).

Toxicity may be detected by a clearing or rather diminution of the background lawn or a reduction in the number of revertants down to a mutation factor of approximately ≤ 0.5 in relation to the solvent control.

The test item was tested in the pre-experiment with the following concentrations: 3.16, 10.0, 31.6, 100, 316, 1000, 2500 and 5000 µg/plate

### Exposure Concentrations

The test item concentrations to be applied in the main experiment were chosen according to the results of the pre-experiment. 5000 µg/plate was selected as the maximum concentration. The concentration range covered two logarithmic decades. The experiment was performed with the following concentrations:
31.6, 100, 316, 1000, 2500 and 5000 µg/plate

As the results of the pre-experiment were in accordance with the criteria of validity, these were reported as a part of the main experiment.

### Experimental Performance

For the plate incorporation method, the following materials were mixed in a test tube and poured over the surface of a minimal agar plate:
100 µL test solution at each dose level, solvent control, negative control or reference mutagen solution (positive control)
500 µL S9 mix(for testing with metabolic activation) or S9 mix substitution buffer (for testing without metabolic activation)
100 µL Bacteria suspension (cf. Preparation of bacteria, pre-culture of the strain)
2000 µL Overlay agar.

Due to clear negative results in the first experiment of this study no independent repetition was performed, as described in 10993-33.

For each strain and dose level, including the controls, three plates were used.

After solidification the plates were inverted and incubated at 37 °C for at least 48 h in the dark.

### Data Recording

The colonies were counted using a ProtoCOL counter (Meintrup DWS Laborgeräte GmbH). If precipitation of the test item precluded automatic counting the revertant colonies were counted by hand. In addition, tester strains with a low spontaneous mutation frequency like TA1535 and TA1537 were counted manually.

### Evaluation of Cytotoxicity

Cytotoxicity can be detected by a clearing or rather diminution of the background lawn (indicated as "N" or "B", respectively in the result tables) or a reduction in the number of revertants down to a mutation factor of approximately ≤ 0.5 in relation to the solvent control.

### Criteria of Validity

A test is considered acceptable if for each strain:
- the bacteria demonstrate their typical responses to ampicillin (TA98, TA100, E. coli WP2 uvrA (pKM101))
- the negative control plates (A. dest.) with and without S9 mix are within the following ranges (mean values of the spontaneous reversion frequency are within the historical control data range (January - December 2020 for all tester strains)): 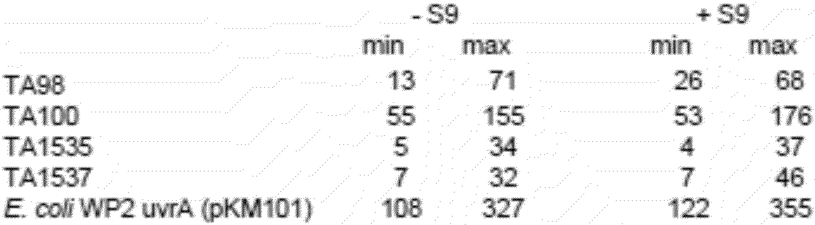
- corresponding background growth on negative control, solvent control and test plates is observed
- the positive controls show a distinct enhancement of revertant rates over the control plate
- at least five different concentrations of each tester strain are analysable.

### Evaluation of Mutagenicity

The Mutation Factor is calculated by dividing the mean value of the revertant counts by the mean values of the solvent control (the exact and not the rounded values are used for calculation).

A test item is considered as mutagenic if:
- a clear and dose-related increase in the number of revertants occurs and/or
- a biologically relevant positive response for at least one of the dose groups occurs in at least one tester strain with or without metabolic activation.

A biologically relevant increase is described as follows:
- if in tester strains TA98, TA100 and E. coli the number of reversions is at least twice as high
- if in tester strains TA1535 and TA1537 the number of reversions is at least three times higher than the reversion rate of the solvent control.

According to OECD guidelines, the biological relevance of the results is the criterion for the interpretation of results, a statistical evaluation of the results is not regarded as necessary.

A test item producing neither a dose related increase in the number of revertants nor a reproducible biologically relevant positive response at any of the dose groups is considered to be non-mutagenic in this system

### Results

### Pre-Experiment

Toxicity may be detected by a clearing or rather diminution of the background lawn or a reduction in the number of revertants down to a mutation factor of approximately ≤ 0.5 in relation to the solvent control.

### Discussion

The test item BIO ACTIVE COLL was investigated for its potential to induce gene mutations according to the plate incorporation test using Salmonella typhimurium strains TA98, TA100, TA1535, TA1537 and tester strain E. coli WP2 uvrA (pKM101).

In the experiment several concentrations of the test item were used. Each assay was conducted with and without metabolic activation. The concentrations, including the controls, were tested in triplicate. The following concentrations of the test item were prepared:
31.6, 100, 316, 1000, 2500 and 5000 µg/plate

No precipitation of the test item was observed in any tester strain used (with and without metabolic activation).

No toxic effects of the test item were noted in any of the five tester strains used up to the highest dose group evaluated with and without metabolic activation.

No biologically relevant increases in revertant colony numbers of any of the five tester strains were observed following treatment with BIO ACTIVE COLL at any concentration level, neither in the presence nor absence of metabolic activation.

The reduction in the number of revertants down to a mutation factor of ≤ 0.5 found in tester strain TA 1537 at a concentration of 316 µg/plate (with metabolic activation) was regarded as not biologically relevant due to lack of a dose-response relationship.

The microbial contamination observed in one plate (TA98, 316 µg/plate, without metabolic activation) did not affect the quality or integrity of the results as the microbial contamination could be clearly distinguished from the Salmonella typhimurium revertants and thus did not affect the evaluation.

All criteria of validity were met.

### Conclusion

In conclusion, it can be stated that during the described mutagenicity test and under the experimental conditions reported, BIO ACTIVE COLL did not cause gene mutations by base pair changes or frameshifts in the genome of the tester strains used.

Therefore, BIO ACTIVE COLL is considered to be non-mutagenic in this bacterial reverse mutation assay.

## Claims

1. A hydrolyzed equine type I collagen powder **characterized in that**:
it has the following particle size distribution:
- at most 10% of the particles have a mean diameter lower than 2.5 microns;
- at least 50% of the particles have a mean diameter lower than 10 microns;
- at least 90% of the particles have a mean diameter lower than 20 microns;
and
it consists of oligopeptides having the following molecular weights:
- at least 30% of the oligopeptide molecules have a weight average molecular weight from 80 to 120 kDa;
- at least 25% of the oligopeptide molecules have a weight average molecular weight from 50 to 70 kDa;
- at least 25% of the oligopeptide molecules have a weight average molecular weight from 25 to 45 kDa;
- at least 85% of said collagen has a molecular weight from 25 to 120 kDa.

2. The collagen powder according to claim 1, **characterized by** the following molecular weights:
- at least 30% of the oligopeptide molecules have a weight average molecular weight from 80 to 120 kDa;
- at least 25% of the oligopeptide molecules have a weight average molecular weight from 50 to 70 kDa;
- at least 25% of the oligopeptide molecules have a weight average molecular weight from 25 to 45 kDa;
- at least 14.5% of the oligopeptide molecules have a weight average molecular weight from 100 to 110 kDa;
- at least 11.5% of the oligopeptide molecules have a weight average molecular weight from 80 to 90 kDa;
- at least 8.0% of the oligopeptide molecules have a weight average molecular weight from 40 to 45kDa;
- at least 85% of said collagen has a molecular weight from 25 to 120 kDa.

3. A pharmaceutical, nutraceutical or cosmetic composition comprising the collagen powder according to claims 1 or 2, together with at least one suitable carrier.

4. The composition according to claim 3, said composition being formulated into gels, creams, ointments, pastes or spray; or said composition being sterile in the form of injectable preparations.

5. Non-therapeutic use of the collagen powder according to claims 1 or 2, as food supplement or as a cosmetic product or as a skin anti-ageing agent.

6. The collagen powder according to claims 1 or 2, for its use in human therapy.

7. A cosmetic product comprising the collagen powder according to claims 1 or 2.

8. A pre-filled syringe comprising the collagen powder according to claims 1 or 2 in the form of a sterile solution.

9. The collagen powder according to claims 1 or 2, for use in the treatment of wounds and sores, by direct application or through medical devices, or sprayed or applied to wounds, burns, sores.

10. The collagen powder according to claims 1 or 2, for use in the treatment of joint disorders by infiltration, optionally packaged as sterile solution in pre-filled syringes.

11. The collagen powder according to claims 1 or 2, for use in the treatment of interstitial cystitis by injection in the form of viscous and/or adhesive compositions.

12. The collagen powder according to claims 1 or 2, for use in the treatment of vulvovaginitis for vaginal application.

13. Non-therapeutic use of the collagen powder according to claims 1 or 2, as a cosmetic filler, for wrinkle smoothing or for solving aesthetic skin problems.

## Patentansprüche

1. Hydrolysiertes equines Typ-I-Kollagenpulver, **dadurch gekennzeichnet, dass** es die folgende Partikelgrößenverteilung aufweist:
- höchstens 10 % der Partikel weisen einen mittleren Durchmesser von weniger als 2,5 Mikrometer auf;
- mindestens 50 % der Partikel weisen einen mittleren Durchmesser von weniger als 10 Mikrometer auf;
- mindestens 90 % der Partikel weisen einen mittleren Durchmesser von weniger als 20 Mikrometer auf;
und
es aus Oligopeptiden mit den folgenden Molekulargewichten besteht:
- mindestens 30 % der Oligopeptidmoleküle weisen ein gewichtsmittleres Molekulargewicht von 80 bis 120 kDa auf;
- mindestens 25 % der Oligopeptidmoleküle weisen ein gewichtsmittleres Molekulargewicht von 50 bis 70 kDa auf;
- mindestens 25 % der Oligopeptidmoleküle weisen ein gewichtsmittleres Molekulargewicht von 25 bis 45 kDa auf;
- mindestens 85 % des Kollagens weist ein Molekulargewicht von 25 bis 120 kDa auf.

2. Kollagenpulver nach Anspruch 1, **gekennzeichnet durch** die folgenden Molekulargewichte:
- mindestens 30 % der Oligopeptidmoleküle weisen ein gewichtsmittleres Molekulargewicht von 80 bis 120 kDa auf;
- mindestens 25 % der Oligopeptidmoleküle weisen ein gewichtsmittleres Molekulargewicht von 50 bis 70 kDa auf;
- mindestens 25 % der Oligopeptidmoleküle weisen ein gewichtsmittleres Molekulargewicht von 25 bis 45 kDa auf;
- mindestens 14,5 % der Oligopeptidmoleküle weisen ein gewichtsmittleres Molekulargewicht von 100 bis 110 kDa auf;
- mindestens 11,5 % der Oligopeptidmoleküle weisen ein gewichtsmittleres Molekulargewicht von 80 bis 90 kDa auf;
- mindestens 8,0 % der Oligopeptidmoleküle weisen ein gewichtsmittleres Molekulargewicht von 40 bis 45 kDa auf;
- mindestens 85 % des Kollagens weist ein Molekulargewicht von 25 bis 120 kDa auf.

3. Pharmazeutische, nutrazeutische oder kosmetische Zusammensetzung, umfassend das Kollagenpulver nach Anspruch 1 oder 2 zusammen mit mindestens einem geeigneten Träger.

4. Zusammensetzung nach Anspruch 3, wobei die Zusammensetzung zu Gelen, Cremes, Salben, Pasten oder Spray formuliert ist; oder wobei die Zusammensetzung steril in Form von injizierbaren Zubereitungen vorliegt.

5. Nicht-therapeutische Verwendung des Kollagenpulvers nach Anspruch 1 oder 2 als Nahrungsergänzungsmittel oder als kosmetisches Produkt oder als Anti-Aging-Mittel für die Haut.

6. Kollagenpulver nach Anspruch 1 oder 2 zur Verwendung in der Humantherapie.

7. Kosmetisches Produkt, umfassend das Kollagenpulver nach Anspruch 1 oder 2.

8. Vorgefüllte Spritze, umfassend das Kollagenpulver nach Anspruch 1 oder 2 in Form einer sterilen Lösung.

9. Kollagenpulver nach Anspruch 1 oder 2 zur Verwendung bei der Behandlung von Wunden und Geschwüren, durch direktes Aufbringen oder mittels Medizinprodukten, oder auf Wunden, Verbrennungen, Geschwüre aufgesprüht oder aufgebracht.

10. Kollagenpulver nach Anspruch 1 oder 2 zur Verwendung bei der Behandlung von Gelenkerkrankungen durch Infiltration, gegebenenfalls als sterile Lösung in vorgefüllten Spritzen verpackt.

11. Kollagenpulver nach Anspruch 1 oder 2 zur Verwendung bei der Behandlung von interstitieller Zystitis durch Injektion in Form von viskosen und/oder adhäsiven Zusammensetzungen.

12. Kollagenpulver nach Anspruch 1 oder 2 zur Verwendung bei der Behandlung von Vulvovaginitis zur vaginalen Anwendung.

13. Nicht-therapeutische Verwendung des Kollagenpulvers nach Anspruch 1 oder 2 als kosmetischer Filler, zur Faltenglättung oder zur Behebung ästhetischer Hautprobleme.

## Revendications

1. Poudre de collagène équin de type I hydrolysé, **caractérisée en ce que** : elle présente la distribution granulométrique suivante :
- au plus 10 % des particules ont un diamètre moyen inférieur à 2,5 microns ;
- au moins 50 % des particules ont un diamètre moyen inférieur à 10 microns ;
- au moins 90 % des particules ont un diamètre moyen inférieur à 20 microns ;
et
il est constitué d'oligopeptides présentant les masses moléculaires suivantes :
- au moins 30 % des molécules d'oligopeptides ont un poids moléculaire moyen en poids compris entre 80 et 120 kDa ;
- au moins 25 % des molécules d'oligopeptides ont un poids moléculaire moyen en poids compris entre 50 et 70 kDa ;
- au moins 25 % des molécules d'oligopeptides ont un poids moléculaire moyen en poids compris entre 25 et 45 kDa ;
- au moins 85 % dudit collagène ont un poids moléculaire compris entre 25 et 120 kDa.

2. Poudre de collagène selon la revendication 1, **caractérisée par** les masses moléculaires suivantes :
- au moins 30 % des molécules d'oligopeptides ont un poids moléculaire moyen en poids compris entre 80 et 120 kDa ;
- au moins 25 % des molécules d'oligopeptides ont un poids moléculaire moyen en poids compris entre 50 et 70 kDa ;
- au moins 25 % des molécules d'oligopeptides ont un poids moléculaire moyen en poids compris entre 25 et 45 kDa ;
- au moins 14,5 % des molécules d'oligopeptide ont un poids moléculaire moyen en poids compris entre 100 et 110 kDa ;
- au moins 11,5 % des molécules d'oligopeptides ont un poids moléculaire moyen en poids compris entre 80 et 90 kDa ;
- au moins 8,0 % des molécules d'oligopeptides ont un poids moléculaire moyen en poids compris entre 40 et 45 kDa ;
- au moins 85 % dudit collagène a un poids moléculaire compris entre 25 et 120 kDa.

3. Composition pharmaceutique, nutraceutique ou cosmétique comprenant la poudre de collagène selon les revendications 1 ou 2, associée à au moins un excipient approprié.

4. Composition selon la revendication 3, ladite composition étant formulée sous forme de gels, de crèmes, de pommades, de pâtes ou de sprays ; ou ladite composition étant stérile sous forme de préparations injectables.

5. Utilisation non thérapeutique de la poudre de collagène selon les revendications 1 ou 2, en tant que complément alimentaire, produit cosmétique ou agent anti-âge pour la peau.

6. Poudre de collagène selon les revendications 1 ou 2, destinée à être utilisée en thérapie humaine.

7. Produit cosmétique comprenant la poudre de collagène selon les revendications 1 ou 2.

8. Seringue préremplie comprenant la poudre de collagène selon les revendications 1 ou 2 sous la forme d'une solution stérile.

9. Poudre de collagène selon les revendications 1 ou 2, destinée à être utilisée dans le traitement des plaies et des lésions, par application directe ou à l'aide de dispositifs médicaux, ou pulvérisée ou appliquée sur des plaies, des brûlures ou des lésions.

10. Poudre de collagène selon les revendications 1 ou 2, destinée à être utilisée dans le traitement des troubles articulaires par infiltration, éventuellement conditionnée sous forme de solution stérile dans des seringues préremplies.

11. Poudre de collagène selon les revendications 1 ou 2, destinée à être utilisée dans le traitement de la cystite interstitielle par injection sous forme de compositions visqueuses et/ou adhésives.

12. Poudre de collagène selon les revendications 1 ou 2, destinée à être utilisée dans le traitement de la vulvovaginite par application vaginale.

13. Utilisation non thérapeutique de la poudre de collagène selon les revendications 1 ou 2, en tant que produit de comblement cosmétique, pour le lissage des rides ou pour résoudre des problèmes esthétiques de la peau.
